(19) Europäisches Patentamt
European Patent Office
Office européen des brevets

(11) EP 1 136 879 A1

(12) EUROPEAN PATENT APPLICATION

(43) Date of publication:
**26.09.2001 Bulletin 2001/39**

(51) Int Cl.[7]: **G03C 5/17**, G03C 5/02, G03C 5/26

(21) Application number: **01105308.9**

(22) Date of filing: **07.03.2001**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **08.03.2000 JP 2000063417**

(71) Applicant: **KONICA CORPORATION**
**Tokyo 163 (JP)**

(72) Inventor: **Suzuki, Tetsuya**
**Hino-shi, Tokyo, 191-8511 (JP)**

(74) Representative:
**Gille Hrabal Struck Neidlein Prop Roos**
**Patentanwälte,**
**Brucknerstrasse 20**
**40593 Düsseldorf (DE)**

(54) **Radiographic imaging system and silver halide photographic material**

(57) An radiographic imaging system for making a radiograph by a radiography apparatus using a photographic combination of a silver halide photographic material having light sensitive layers on both sides of the support and an intensifying screen is disclosed, wherein the photographic material exhibits a cross-over of not more than 15%, a specified point gamma value within the specified density range on the characteristic curve when the photographic combination is exposed to X-ray, and wherein the radiography apparatus conducts making a radiograph under the condition that a distance between a focal point of an X-ray tube and the photographic combination is 1.0 to 3.0 m and a distance between an object and the photographic combination is 0.2 to 1.5 m.

EP 1 136 879 A1

## Description

### FIELD OF THE INVENTION

The present invention relates to a radiographic imaging system and a silver halide photographic light sensitive material, thereby achieving enhanced image quality, and in particular to a silver halide photographic light sensitive material exhibiting superior image quality and/or enhanced sensitivity and a radiographic image forming system, whereby images exhibiting high speed and superior sharpness are obtained in the field of radiographic diagnosis of bone and stomach.

### BACKGROUND OF THE INVENTION

In medical radiography, tissue images of a patient can be obtained using a photographic material having on a transparent support a light sensitive silver halide emulsion layer and on which recording a transmission type pattern is recorded. The radiographic transmission pattern can be recorded using only a silver halide photographic material. Exposure of a human body to a large amount of X-rays is not desirable and therefore X-ray photography is conducted by having the silver halide photographic material combined with a X-ray intensifying screen (which is also called a fluorescent screen or simply a screen). The X-ray intensifying screen is provided with a phosphor on the support and the phosphor layer absorbs X-rays and converts them to visible light to which the silver halide photographic material is highly sensitive, thereby leading to enhancement of sensitivity of the X-ray photography system.

To enhance sensitivity of the medical radiography system, a silver halide photographic material having light sensitive silver halide emulsion layers on both sides thereof (hereinafter, also denoted as a photographic material or a film), is subjected to X-ray photography, while both sides thereof are laminated with a X-ray intensifying screen. Such a combination of the silver halide photographic material and the intensifying screen for use in X-ray photographing is also called a photographic combination and a system employing such a combination is also called a screen-film system. In silver halide photographic materials having silver halide emulsion layers on both sides of the support, however, there have been problems such that deterioration in image quality, caused by cross-over light easily occurs. The cross-over light is visible light which is emitted from the intensifying screen arranged on one side of the photographic material and transits through an about 180 μm thick support to a light sensitive layer on the other side, resulting in lowering of image quality and specifically, deterioration in sharpness.

Radiographic image forming methods which have been developed for the purpose of radiographic diagnosis of bone and stomach have not yet reached a radiography system having sufficiently satisfactory image quality and enhanced sensitivity. Regarding radiographic images of bone, it is very important for diagnosis that the fine structure of bone can be explicitly observed, and in radiographic images of stomach, it is important that the double-contrast image of gastric mucous membrane structure can be clearly observed, but currently, these do not reach satisfactory levels.

In bone radiography, images of bone which is small in an X-ray transmission and a surrounding soft tissue which is large in X-ray transmission must be simultaneously finished to an easily observable density level. In cases when a relatively low contrast photography system is applied, easily observable images can be formed on the whole but it becomes difficult to observe the fine structure of bone. To the contrary, in cases when a relatively high contrast photography system is applied, the fine structure of bone can be definitely observed but details of soft tissue become totally dead black and details thereof cannot be observed on the viewing box.

In stomach radiography, it is difficult to simultaneously observe the fine structure of gastric mucous membrane adhered with a barium contrast medium and also the upper portion of the stomach body which is filled with gas, through a one-shot photograph. The X-ray transmission depends of portions of the stomach so that meeting the requirement for a broad photographic latitude is difficult to be consistent with clear observation of the fine structure.

Sharpness can be enhanced by increasing contrast of a photographic material but graininess is deteriorated at the same time. Observation of the fine structure of bone and gastric mucous membrane and detection of low-contrasted tumors are dispensable at the same time but deteriorated graininess accompanied by enhancement of sharpness results in a lowering of detectability of a low-contrasted image. Accordingly, there has been desired a radiographic image forming process applicable to stomach and bone radiography, exhibiting superior sharpness without deteriorating graininess and a silver halide photographic material for use thereof.

### SUMMARY OF THE INVENTION

It is therefore an object of the present invention to provide a silver halide photographic material exhibiting superior sharpness without deteriorating graininess and a radiographic image forming system using the same, for use in stomach and bone radiography.

Specifically, it is an object of the invention to provide a radiographic intensifying screen-film system exhibiting superior sharpness suitable for magnification radiography and enhanced sensitivity suited for use in bone and stomach radiography.

The object of the present invention can be accomplished by the following constitution:

(1) an radiographic imaging system for making a radiograph by means of a radiography apparatus using a photographic combination of a silver halide photographic light sensitive material in combination with at least an intensifying screen, the photographic material comprising a support having a light sensitive silver halide emulsion layer on each of both sides of the support, wherein the photographic material exhibits a cross-over of not more than 15%, a point gamma of 1.8 to 3.0 within the range of a density of 0.7 to 1.5 on a characteristic curve and a point gamma of 1.2 to 2.0 within the range of a density of 2.0 to 2.8 on the characteristic curve when the photographic combination is exposed to X-ray, and wherein the radiography apparatus conducts making a radiograph under the condition that a distance between a focal point of an X-ray tube and the photographic combination is 1.0 to 3.0 m and a distance between an object and the photographic combination is 0.2 to 1.5 m; and

(2) a silver halide photographic light sensitive material which is combined with at least an intensifying screen to form a photographic combination for use in a radiographic imaging system for making a radiograph by a radiography apparatus, wherein the photographic material comprises a support having a light sensitive silver halide emulsion layer on each of both sides of the support, the photographic material exhibiting a cross-over of not more than 15%, a point gamma of 1.8 to 3.0 within the range of a density of 0.7 to 1.5 on a characteristic curve and a point gamma of 1.2 to 2.0 within the range of a density of 2.0 to 2.8 on the characteristic curve when the photographic combination is exposed to X-ray and subjected to processing (A), wherein the processing (A) is conducted using a roller transport type automatic processor and the following developer solution (D) and fixed solution (F) at a developing temperature of 35° C for a developing time of 12 to 15 sec. and at a fixing temperature of 33° C for a fixing time of 7 to 13 sec.:

| Developer solution D | |
|---|---|
| Water | 800 ml |
| Potassium hydroxide | 17.1 g |
| Potassium sulfite | 65.7 g |
| Potassium metabisulfite | 6.7 g |
| Hydroquinone | 27.0 g |
| Potassium carbonate | 9.0 g |
| Trisodium ethylenediaminetetraacetate (35% aqueous solution) | 8.0 g |
| Triethylene glycol | 11.8 g |
| 5-nitroindazole | 0.03 g |
| Acetic acid (80% aqueous solution) | 16.0 g |
| 1-Phenyl-3-pyrazolidone | 1.2 g |
| 1-Phenyl-5-mercaptotetrazole | 0.015 g |
| 5-Methylbenzotriazole | 0.024 g |
| Glutar aldehyde (50% aqueous solution) | 8.6 g |
| Potassium bromide | 2.6 g |
| Water to make | 1 lit. |

in which the pH is adjusted to 10.33; and

| Fixer solution F | |
|---|---|
| Water | 130 ml |
| Ammonium thiosulfate (75% weight/volume) | 245.5 g |
| Sodium sulfite | 11.7 g |
| Sodium hydroxide | 6.56 g |
| Sodium gluconate | 2.02 g |
| Citric acid monohydrate | 2.40 g |
| Acetic acid (80% aqueous solution) | 22.5 g |
| Aluminum sulfate | 9.8 g |

(continued)

| Fixer solution F | |
|---|---|
| 75% Sulfuric acid | 5.73 g |
| Water to make | 1 lit. |

in which the pH is adjusted to 4.18.

## BRIEF EXPLANATION OF THE DRAWING

Fig. 1 is a schematic illustration of an edge effect achieved through refraction of an X ray.
Fig. 2 is an example of radiographic imaging systems of the invention.
Fig. 3 shows a characteristic curve and a gamma curve of a photographic material of the invention.

## DETAILED DESCRIPTION OF THE INVENTION

To enhance detectability in radiography, besides contrast-increasing of the silver halide photographic material, the fine structure of bone and gastric mucous membrane can be detected to further smaller size tissue levels by applying magnification radiography. In this method, the same effect as an enhancement of sharpness can be achieved without deteriorating graininess. However, magnification radiography results in blurring of images due to geometrical non-sharpness. This blurring is non-sharpness depending on the focus size of the X ray tube (3) and the magnification factor. Usually, the smaller the focus of the X-ray tube, the sharper image is, but the output capacity is limited so that the photographing range is also limited. A smaller focus size is desirable to enhance representation of the fine structure of bone and gastric mucous membrane and the focus size number is generally within the range of 0.6 to 1.2 mm.
This blurring can be overcome by the refraction contrast imaging technology in this invention. In cases when this imaging technology is applied to bone and stomach radiography, the distance between the X ray tube and a photographic combination (or screen-film system) needs to be increased, so that the photographic combination exhibiting high image quality and high sensitivity is needed to overcome the foregoing problem. Thus, enhancement of detectability of the fine structure, without deteriorating detectability of any tumors can be achieved by application of magnification radiography with a high speed photographic combination (screen-film system), without deteriorating the image quality.
Accordingly, it is an object of this invention to provide a high speed photographic combination (or screen-film system) for use in bone and stomach radiography to conduct magnification radiography of enhanced image quality and an X ray imaging method by the use of the same, as specifically detailed below.
X-rays are electromagnetic waves and have properties of a wave. When an X ray beam penetrates objects differing in their refractive index, refraction occurs at the interface thereof. As is schematically shown in Fig. 1, in the X ray transmission image corresponding to the interface differing in refractive index and projected on the X ray detector (which corresponds to photographic combination "5" comprised of a photographic material and an intensifying screen in Fig. 1), a portion in which the X ray density is decreased by refraction of the X-ray and a portion in which the X-ray density is increased by overlapping of a refracted X-ray beam with a rectilinear X-ray beam are produced, resulting in an edge-enhanced image. Such a phenomenon is called a refraction contrast. This behavior is shown in Fig. 1, wherein "4" represents an object, "7" represents X-rays, "8" represents an X-ray intensity distribution to which the photographic combination (5) is exposed, and "9" represents an X-ray intensity. It is shown that the edge-enhancement effect is achieved at the interface, due to refraction of X-rays. Conventional X-ray images exhibit only an absorption contrast based on the difference in absorption and such a refraction contrast has not been fully employed so far. In the invention, even when blurring of an image is caused by a penumbra in magnification radiography, such blurring can be overcome by applying the refraction contrast to cause edge enhancement, leading to a magnified X ray image exhibiting superior sharpness.
In this invention, a screen-film system for use in bone and stomach radiography, exhibiting higher sensitivity is preferable. However, the use of an intensifying screen exhibiting higher sensitivity (or luminance) for enhancing the sensitivity of the system deteriorates sharpness, leading to a lowering of detectability of the fine structure of bone or gastric mucous membrane. It is therefore preferable to enhance sensitivity of a silver halide photographic material. To enhance the sensitivity, it is necessary to increase the silver halide grain size. However, there are problems that an increase of the grain size results in a lowering of covering power, i.e., unless the coating amount of silver halide grains per unit area is increased, the maximum density or contrast is lowered, leading to deteriorated graininess.
In conventional bone and stomach radiography systems, in which the distance between an X-ray source (i. e., focus of an X-ray tube) and a screen-film (or a photographic combination) is within the range of 0.5 to 2.0 m, there

has been a problem that X-rays are radially emitted from the X-ray source so that when a photographic material comprising a support having on both sides thereof, emulsion layers is exposed, displacement occurs between images of the both sides, at the circumferential portions, leading to a lowering of sharpness. In the radiography system according to this invention, however, the distance between the X-ray source and the screen-film is made longer than that of the conventional radiography system, so that X-rays emitted from the X-ray source becomes a state close to being in parallel from being radial (in other words, from diffuse radiation to specular radiation), reducing displacement between images on both sides to reduce a lowering of sharpness. Further, a lowering of sharpness can be prevented by decreasing cross-over light to levels of not more than 15%. In preferred embodiments of radiography of this invention, the distance between the focus of an X-ray source, such as an X-ray tube and a photographic combination is preferably 1.0 to 3.0 m, and more preferably 1.5 to 2.5 m. The distance between an object (i.e., the center of an object which the X-rays penetrate) and a photographic combination is preferably 0.2 to 1.5 m, and more preferably 0.5 to 1.2 m.

Fig. 2 illustrates an example of a radiography system of this invention. In Fig. 2, "3" represents an X-ray tube as an X-ray source; $R_1$ represents the distance between focal position (10) of X-ray tube (3) and central line (13) of the object (4) and the object (4) is arranged so as to vary the distance ($R_1$) by moving on distance-marked support (12); photographic material (11) of photographic combination (5) which is comprised of the photographic material and an intensifying screen is arranged, distance $R_2$ apart from the central line (13) of the object (4).

In this invention, photographic materials are preferably processed according to processing (A). Thus, using a roller transport type automatic processor, the processing (A) is conducted at a developing temperature of 35° C for a developing time of 12 to 15 sec. and at a fixing temperature of 33° C for a fixing time of 7 to 13 sec. For example, processing is carried out over a period of 45 sec. with developer solution D and fixer solution F using automatic processor SRX-502, available from Konica Corp., in which the developing time is 13.5 sec., the fixing time is 9.5 sec., the washing time is 9.5 sec. and the squeezing and drying time is 12.5 sec. The developing time refers to the period of time of from the time when the top of a photographic material is immersed into a developer solution to the time when the top is immersed into a fixer solution, and the fixing time refers to the period of time of from the time when the top of the photographic material is immersed into the fixer solution to the time when the top is immersed into a washing water. If a silver halide photographic material which has been processed according to the foregoing conditions exhibits a characteristic curve meeting the requirements of this invention, the photographic material is therefore regarded to fall within the scope of this invention.

The developer solution D and fixer solution F are as follow:

| Developer solution D | |
|---|---|
| Water | 800 ml |
| Potassium hydroxide | 17.1 g |
| Potassium sulfite | 65.7 g |
| Potassium metabisulfite | 6.7 g |
| Hydroquinone | 27.0 g |
| Potassium carbonate | 9.0 g |
| Trisodium ethylenediaminetetraacetate (35% aqueous solution) | 8.0 g |
| Triethylene glycol | 11.8 g |
| 5-nitroindazole | 0.03 g |
| Acetic acid (80% aqueous solution) | 16.0 g |
| 1-Phenyl-3-pyrazolidone | 1.2 g |
| 1-Phenyl-5-mercaptotetrazole | 0.015 g |
| 5-Methylbenzotriazole | 0.024 g |
| Glutar aldehyde (50% aqueous solution) | 8.6 g |
| Potassium bromide | 2.6 g |

and water is added to make the total amount 1 liter and the pH is adjusted to 10.33; and

| Fixer solution F | |
|---|---|
| Water | 130 ml |
| Ammonium thiosulfate (75% weight/volume) | 245.5 g |
| Sodium sulfite | 11.7 g |
| Sodium hydroxide | 6.56 g |

(continued)

| Fixer solution F | |
|---|---|
| Sodium gluconate | 2.02 g |
| Citric acid monohydrate | 2.40 g |
| Acetic acid (80% aqueous solution) | 22.5 g |
| Aluminum sulfate | 9.8 g |
| 75% Sulfuric acid | 5.73 g |

and water is added to make the total amount 1 liter, and the pH is adjusted to 4.18.

In silver halide photographic material comprising a transparent support having emulsion layers on both sides of the support, cross-over exposure occurs when light emitted by one screen passes through the adjacent emulsion layer and the support to imagewise expose the emulsion layer on the opposite side of the support. Thus, cross-over light is light which has crossed over. Percent cross-over [denoted as cross-over (%)] can be determined according to the method described in T.I. Abbott et al. U.S. Patent No. 4,425,425. Thus, in a silver halide photographic material having two substantial identical light sensitive layers on opposite sides of a transparent support, an intensifying screen, the photographic material and black opaque paper are arranged in this order from an X-ray source, which are packed in a cassette for use in radiography and subjected to stepwise exposure to X-rays. After completion of processing, an image of the light sensitive layer in contact with the screen is separated from the image of the light sensitive layer on the opposite side and characteristic curves for images of the two light sensitive layers are respectively obtained. The difference in sensitivity between the linear portions of the characteristic curves is denoted as $\Delta$log E, and percent cross-over is defined as below:

Cross-over (%) = 100/[antilog ($\Delta$log E) + 1].

A lower cross-over percent results in sharper images. There are various techniques for reducing the cross-over and one preferred method is to fix a dye capable of decolorizing upon processing between the support and the light sensitive layer. As described in U.S. Patent No. 4,803,150, the use of a dye in the form of micro-crystals results in improved fixation, improved decolorizability and a larger content of the dye, thereby leading to reduced crossover. This method causes no loss of sensitivity due to insufficient fixation and enables decolorization of the dye in processing for 90 sec., leading to a cross-over of not more than 15%. In the dye layer for reducing cross-over, it is preferred to arrange a dye as closely as possible. It is therefore preferred to reduce the coating amount of gelatin used as a binder to make the thickness of the dye layer 0.5 or less. However, an extremely thin dye layer results in troubles in adhesion and thus, the dye layer thickness is preferably 0.05 to 0.3 $\mu$m.

Reduction of the cross-over can also achieved by increasing the thickness of the silver halide emulsion layer or coloring the support.

The radiographic image forming method exhibiting a characteristic curve within the range of this invention provides images which can be easily diagnosed for of bone and stomach. The point gamma of 1.8 to 3.0 between densities of 0.7 and 1.5 is a relatively high contrast so that in bone radiographs, contrast within the low and intermediate density regions is enhanced, making the bone skeleton clear; the point gamma of 1.2 to 2.0 between densities of 2.0 and 2.8 is a relatively low contrast so that the latitude in the high density region is broadened and details of soft tissue are not made entirely dead black, forming easily diagnosable images of bone tissue and soft tissue through a one shot radiograph. Similarly, even in stomach radiographs, images are formed without forming dead black portions, whereby the fine structure of the gastric wall can be clearly observed.

The point gamma is defined as the slope of a tangential line on a characteristic curve represented in orthogonal coordinates of the optical density (y-axis) and common logarithmic exposure (x-axis), each having an identical unit length. Thus, when an angle between the tangential line and the x-axis is denoted as $\theta$, the point gamma is represented by a value of tan $\theta$. Characteristic curve (1) and its differential curve, i.e., $\gamma$(gamma)-curve (2) are shown in Fig. 3, wherein D represents the optical density and Log E represents the logarithmic exposure.

Silver halide photographic materials exhibiting a characteristic curve meeting requirements of this invention can be obtained by various methods. For example, two emulsions different in sensitivity are selected, the sensitivity ratio of which is preferably within the range of 1:0.3 to 1:0.7. The two emulsions may be mixed and coated, or coated separately. One preferred embodiment is an arrangement comprised of the upper layer of a high-speed emulsion and the lower layer of a low-speed emulsion. The ratio of low-speed emulsion to high-speed emulsion, based on silver is preferably 0.7 to 1.0, and more preferably 0.5 to 0.2.

A silver halide emulsion used in silver halide photographic materials relating to this invention is preferably one which is comprised of tabular silver halide grains. The silver halide tabular grain emulsion exhibits balanced relationship between sensitivity and granularity, superior spectral sensitization characteristics, and enhanced capability of reducing cross-over.

A silver halide photographic material used in this invention preferably contains at least two silver halide tabular grain emulsions each which are substantially identical in average circular equivalent diameter. The expression "substantially identical in average circular equivalent diameter" means that the ratio of a larger average circular equivalent diameter to a smaller average circular equivalent diameter is not more than 1.15, and preferably not more than 1.05.

The silver halide photographic material used in this invention preferably has, on al least one side of the support, at least two light sensitive silver halide emulsion layers containing tabular silver halide grains, which are different and identical in average circular equivalent diameter. In at least two tabular grain emulsions which are identical in average circular diameter and different in average grain thickness, the ratio of a larger average grain thickness (B) to a smaller average grain thickness (A), i.e., B/A is preferably 1.05 to 20.0, more preferably 1.10 to 15.0, and still more preferably 1.15 to 10.0.

Tabular silver halide grains contained in the silver halide photographic material used in this invention are preferably doped with at least one selected from the group of ions and atoms of gallium, indium, elements of the 8th group, 9th group and 10th group of the periodical table, and their complexes.

The tabular silver halide grains used in this invention preferably have a major face comprising {100} face and {111} face. The tabular grains preferably have an average circular equivalent diameter of 0.2 to 6.0 μm, and more preferably 0.2 to 3.0 μm. In this invention, the circular equivalent diameter refers to a diameter of a circle having the same area as the projected area of the major face of the tabular grain. The tabular grains used in this invention preferably exhibit an average value of a ratio of circular equivalent diameter to thickness (which is denoted as aspect ratio) of not less than 2.0, more preferably 2.0 to 20.0, and still more preferably 4.0 to 15.0. At least 100 grains are measured to determine the average aspect ratio. The average thickness of the tabular grains is preferably 0.03 to 0.5 μm, and more preferably 0.03 to 0.3 μm. The thickness of a tabular silver halide grain is defined as a distance between major faces, i.e., to the minimum distance between two parallel crystal faces having the largest area. The thickness of tabular grains can be determined from shadowed electron micrographs of silver halide grains, prepared by the carbon replica process, or electron micrographs of the section of a silver halide emulsion coated on a support. The tabular silver halide grains used in this invention may have a crystal face other than the major faces.

The tabular silver halide grain emulsion used in this invention is preferably monodisperse and a coefficient of variation of circular equivalent diameter is preferably not more than 20%. The tabular silver halide grain emulsion may be mixed with a monodisperse tabular grain emulsion having a different circular equivalent diameter, a polydisperse tabular grain emulsion having a broad grain size distribution, an emulsion comprised of regular crystal grains such as cubic, octahedral, or tetradecahedral grains or a multiple twin crystal grain emulsion comprised of tabular grains having multiple-twinned planes, within the range of affecting no effect on this invention. Herein, the coefficient of variation is a value (%) of a fluctuation (standard deviation) of circular equivalent diameter divided by an average circular equivalent diameter.

The tabular silver halide grains used in this invention is not specifically limited with respect to halide composition, and are preferably silver chloride, silver bromide, silver chlorobromide, or silver iodobromide. The average iodide content is preferably not more than 3.0 mol%, and more preferably not more than 1.0 mol%. The tabular silver halide grains may be homogeneous with respect to halide composition within the grain, or may be core/shell type grains internally containing an iodide-localized portion or grains having a high iodide portion in the vicinity of the grain surface.

Preparation of tabular grains having {100} major faces is referred to U.S. Patent No. 4,063,951, 4,386,156, 5,275,930, and 5,314,798. The size and the shape of tabular silver halide grains can be controlled by adjusting a temperature, a pAg (or pBr, PCl), a pH, and flow rates of silver salt and halide salt solutions at the time of grain formation. The pAg at the time of forming tabular silver halide grains is preferably 5.0 to 9.0. The average iodide content of tabular silver halide grains can be controlled by varying the halide composition of the halide salt solution. Silver halide solvents such as ammonia, thioethers and thioureas may be employed at the time of preparation of tabular grains. The silver halide emulsion may be any one of a surface latent image type forming latent images on the grain surface, internal latent image type forming latent images in the interior of the grain and a surface and internal latent image type.

In silver halide grains relating to this invention, metals of gallium, indium, 8th group elements (Fe, Ru, Os), 9th group elements (Co, Rh, Ir) and 10th group elements (Ni, Pd, Pt) may be doped in the form of an atom, an ion or a complex ion. In one preferred embodiment are employed a hexa-coordinate metal complex represented by the following formula, as a dopant:

$$[MeL_6]^n$$

wherein Me represents a polyvalent metal ion having a filled frontier orbital, and preferably $Fe^{2+}$, $Ru^{2+}$, $Os^{2+}$, $Co^{3+}$, $Rh^{3+}$, $Ir^{3+}$, $Pd^{4+}$ or $Pt^{4+}$; $L_6$ represents six coordinate ligands which can be independently selected, provided that at least four of the ligands are anion ligands and at least one (preferably at least three, and more preferably at least four) of the ligands exhibit(s) a higher electronegativity than a halogen ligand; and n represents 2-, 3- or 4-. $L_6$ is preferably

ligands containing one to six $CN^-$.

Examples of dopants are shown below:

| | |
|---|---|
| SET-1 | $[Fe(CN)_6]^{4-}$ |
| SET-2 | $[Ru(CN)_6]^{4-}$ |
| SET-3 | $[Os(CN)_6]^{4-}$ |
| SET-4 | $[Rh(CN)_6]^{3-}$ |
| SET-5 | $[Ir(CN)_6]^{3-}$ |
| SET-6 | $[Fe\cdot hydrazine(CN)_5]^{4-}$ |
| SET-7 | $[RuCl(CN)_5]^{4-}$ |
| SET-8 | $[OsBr(CN)_5]^{4-}$ |
| SET-9 | $[RhF(CN)_5]^{4-}$ |
| SET-10 | $[IrBr(CN)_5]^{3-}$ |
| SET-11 | $[OsBr(CN)_5]^{3-}$ |
| SET-12 | $[RuF_2(CN)_4]^{4-}$ |
| SET-13 | $[OsCl_2(CN)_4]^{4-}$ |
| SET-14 | $[RhI_2(CN)_4]^{4-}$ |
| SET-15 | $[IrBr_2(CN)_4]^{4-}$ |
| SET-16 | $[Ru(CN)_5(OCN)]^{4-}$ |
| SET-17 | $[Ru(CN)_5(N_3)]^{4-}$ |
| SET-18 | $[Ru(CN)_5(SCN)]^{4-}$ |
| SET-19 | $[Rh(CN)_5(SeCN)]^{4-}$ |
| SET-20 | $[Ir(CN)_5(HOH)]^{4-}$ |
| SET-21 | $[Fe(CN)_3Cl_3]^{3-}$ |
| SET-22 | $[Ru(CO)_2(CN)_4]^{2-}$ |
| SET-23 | $[Os(CN)Cl_5]^{4-}$ |
| SET-24 | $[Co(CN)_6]^{3-}$ |
| SET-25 | $[Ir(CN)_4\cdot oxalate]^{3-}$ |
| SET-26 | $[In(NCS)_6]^{3-}$ |
| SET-26 | $[Ga(NCS)_6]^{3-}$ |

Further, there may be applied oligomer coordinate complexes taught in Evans, U.S. Patent No. 5,024,931 to enhance speed (sensitivity). Dopants are effective in usual concentrations (which is finally a concentration, based on silver of total tabular silver halide grains). A dopant forming a shallow electron trap is occluded preferably in an amount of $1\times10^{-7}$ to a solubility limit per mol of silver (exemplary, $5\times10^{-4}$ mol per mol of silver), and more preferably $1\times10^{-7}$ to $1\times10^{-4}$ mol per mol of silver.

The above-mentioned metal compounds to be occluded in tabular silver halide grains are added through solution in water or an appropriate solvents such as methanol or acetone. The metal compound may be in the form of salts such a potassium salt. Occlusion of the metal compound can be detected in such a manner that the emulsion is separated by centrifugation and the supernatant solution ot separated grains are subjected to atomic absorption spectrophotometry to determine the metal contents thereof. There may be added an aqueous hydrogen halide (HCl, HBr, etc.) od aqueous alkali halide (KCl, KBr, NaBr, etc.) to stabilize the solution. The metal compound may be added to a reaction vessel prior to grain formation or incorporated during grain formation. The metal compound may be mixed with an aqueous silver salt (e.g., $AgNO_3$) solution or an aqueous halide salt (e.g., NaCl, KBr, KI) solution and continuously added during formation of silver halide grains. Further, the metal compound is dissolved in a solution independently from the silver salt and alklali halide solutions and continuously added at a time during grain formation. Furthermore, the combination of such adding method as described above may also be employed.

The emulsion relating to this invention may be subjected to desalting to removing soluble salts (desalting process), employing noodle washing or flocculation washing. Examples of preferred washing include a technique of using a sulfo group-containing aromatic hydrocarbon type resin described in JP-B No. 35-16086 (hereinafter, the term, JP-B means published Japanese Patent) and a technique of using polymeric coagulants such as G3 and G8 exemplified in JP-A 63-158641 (hereinafter, the term, JP-A means an unexamined, published Japanese Patent Application).

Examples of hydrophilic colloids used in this invention include gelatin derivatives, graft copolymers of gelatin and other polymers, proteins such as albumine and casein, cellulose derivatives such as hydroxycellulose, carboxymethyl cellulose and cellulose sulfuric acid ester, sodium alginate, saccharide derivatives such as starch derivatives, and

synthetic hydrophilic polymers such as polyvinyl alcohol, polyvinyl alcohol partial acetal, poly-N-vinyl pyrrolidine, poyacrylic acid, polymethacrylic acid, polyacrylamide, polyvinyl imidazole, and polyvinyl pyrazolo, including homopolymers and copolymers. Gelatin hydrolystes or gelatin enzymatic degradation products are also usable. As described in U.S. Patent No. 4,713,323, low methionine content gelatin (containing methionine of less than 30 μg and specifically less than 12 μg per g of gelatin) is also preferably employed in the formation of tabular silver halide grains.

Silver halide emulsions used in this invention are suitably subjected to noble metal sensitization or chalcogen sensitization at a pH of 4.0 or more. The preferred pH range is 4.0 to 10.0, and more preferably 5.0 to 8.0. The noble metal sensitization is preferably gold sensitization, in which gold compounds, and gold complexes such as gold-thiocyanate compounds are employed. There may be employed complexes of metals other than gold, including platinum, iridium, osmium, palladium, rhodium, and ruthenium.

Examples of gold sensitizer include a gold chloroaurate, a gold thiourea complex, potassium chloroaurate, auric trichloride, potassium auric thiocyanate, potassium iodoaurate, tetracyanoauric amide, ammonium aurothiocyanate, and pyridyl trichlorogold. The addition of these gold sensitizers depending of various conditions is preferably in an amount of $5\times10^{-7}$ to $5\times10^{-3}$ mol per mol of silver halide, and more preferably $2\times10^{-6}$ to $4\times10^{-4}$. Examples of sulfur sensitizers include thiosulfates, allythiocarbamido-thiourea, allyl thioisocyanate, cystine, p-toluenethiosulfonate and sulfur sensitizers described in U.S. Patent No. 1,574,944 and 3,656,955, German Patent No. 1,422,869, JP-B 56-24937, and JP-A 55-45016. The sulfur sensitizer is incorporated in amounts sufficient to effectively increase sensitivity. The amount is variable according to various conditions such as silver halide grain sizes and generally $5\times10^{-8}$ to $5\times10^{-5}$ mol per mol of silver halide.

Selenium sensitizatization and/or tellurium sensitization are also preferably employed. Compounds commonly known as a selenium sensitizer are applicable. Selenium sensitization is usually conducted by adding a labile-type selenium compound and/or a non-labile-type selenium compound at a relatively high temperature, e.g., 40° C or higher, with stirring for a given period of time. As a labile selenium compound are employed compounds described in JP-B No. 44-15748 and 43-13489 and JP-A No. 2-130976. Examples of labile selenium sensitizers include isoselenocyanates (e.g., aliphatic isoselenocyanates such as allylisoselenocyanate), selenoureas, selenoketones, selenoamides, selenocarboxylic acids (e.g., 2-selenopropionic acid, 2-selenobutyric acid), selenoesters, diacylselenides (e.g., bis-3-chloro-2,6-dimethoxybenzoyl selenide). selenophosphates, phosphine selenides, and colloidal metallic selenium. Labile-type selenium compounds are not limited to the compounds described above. Usable as a non-lable-type selenium compound are compounds described in JP-B 46-4553, 53-34492 and 52-34491. Exemplary examples thereof include selenious acid, potassium selenocyanate, selenazoles and their quaternary salts, diaryl selenide, diaryl diselenide, dialkyl selenide, dialkul diselenide, 2-selenazolidine-dione, 2-selenooxazolidine^thione, and their derivatives. The amount of a selenium sensitizer to be added at the time of chemical sensitization, depending of a selenium compound, silver halide grains, and chemical sensitization conditions is preferably not less than $1\times10^{-8}$ mol per mol of silver halide, and more preferably $1\times10^{-7}$ to $1x10^{-3}$. The selenium sensitizer is added through solution in water or an optimal solvent such as ethanol, by pre-mixing with an aqueous gelatin solution, or in the form of a dispersion emulsified in an organic solvent-soluble polymer, as described in JP-A 4-140739. In this invention, tellurium sensitization is also applicable as chemical sensitization. Examples of preferred tellurium sensitizers are described in U.S. Patent No. 3,772,031, British patent No. 235,211, Canadian Patent No. 800,958, J. Chem. Soc. Chem. Commun. 635 (1980), ibid 1102 )1979), ibid 645 (1979), and J. Chem. Soc. Perkin Trans., 1, 2191 (1988).

Silver halide emulsions used in this invention can also be spectrally sensitized. As a spectrally sensitizing dyes usable in this invention are methine dyes, including cyanine dyes, merocyanine dyes, complex cyanine dyes, complex merocyanine dyes, holopolar cyanine dyes, hemicyanine dyes, styryl dyes and hemioxonol dyes. The addition of a sensitizing dye, depending on the kind thereof and emulsion conditions is preferably in an amount of 10 to 900 mg per mol of silver halide, and more preferably 60 to 400 mg. The sensitizing dye is added preferably before completion of chemical ripening, in which addition may be separated to a few times. The addition is more preferably after completion of grain growth and before completion of chemical sensitization, and still more preferably before staring chemical ripening.

Taking into account of emulsion stability, a chamical ripening-stopping agent is preferably used to stop chemical sensitization (chemical ripening). The chemical ripeing-stopping agent include, for example, a halide compound (e.g., potassium bromide, sodium chloride), and organic compounds known as an antifoggant or stabilizer (e.g., 4-hydroxy-6-methyl-1,3,3a,7-tetrazaindene). These compounds are used alone or in combination.

A variety of adjuvants may be incorporated to the photographic material in accordance with its purpose. The adjuvants are described in Research Disclosure (RD) 17643 (Dec., 1978), ibid 18716 (Nov., 1979), and ibid 308119 (Dec., 1989). Kinds of compounds described in these RD and described section are shown below.

Table 1

| | RD-17643 | RD-18716 | RD-308119 |
|---|---|---|---|
| Additive | Page Sec. | Page | Page Sec. |
| Chemical sensitizer | 23 III | 648 upper right | 996 III |
| Sensitizing dye | 23 IV | 648-649 | 996-8 IVA |
| Desensitizing dye | 23 IV | | 998 VB |
| Dye | 25-26 VIII | 649-650 | 1003 VIII |
| Developing accelerator | 29 XIII | 648 upper right | |
| Antifoggant/stabilizer | 24 IV | 649 upper right | 1006-7 VI |
| Brightening agent | 24 V | | 998 V |
| Hardening agent | 26 X | 651 left | 1004-5 X |
| Surfactant | 26-27 XI | 650 right | 1005-6 XI |
| Antistatic agent | 27 XII | 650 right | 1006-7 XIII |
| Plasticizer | 27 XII | 650 right | 1006 XII |
| Slipping agent | 27 XII | | |
| Matting agent | 28 XVI | 650 right | 1008-9 XVI |
| Binder | 26 XXII | | 1003-4 IX |
| Support | 28 XVII | | 1009 XVII |

Supports usable in photographic materials of this invention are described in RD 17643, page 28 and RD 308119, page 1009.

Representative constitution of a silver halide photographic material used in this invention comprises on both side (front and rear side) of a blue-tinted, transparent support, a sublayer, a dye layer to reduce cross-over, at least a light sensitive silver halide emulsion layer and a protective layer in the order described. The layer on both side (or front and rear sides) are preferably substantially identical to each other.

The support is formed of a transparent material such as polyethylene terephthalate, which is blue-tinted. Various blue dyes are employed, such as anthraquinone type dyes, known as a dye for use in radiographic films. The support thickness is optimally selected from the range of 160 to 200 μm. Similarly to conventional radiographic films, a sublayer comprised of an aqueous soluble polymer such as gelatin is provided on the support.

There is provided a dye layer on the sublayer to reduce cross-over. The dye layer is formed as a colloidal layer containing a dye and preferably is capable of being decolored in the processing afore-mentioned. A dye is preferably fixed in the lower portion of the layer so that the dye does not diffuse to the upper light sensitive silver halide emulsion layer of the protective layer. The light sensitive silver halide emulsion layer is provided on the dye layer. The silver halide emulsion contained in the layer can be prepared according to commonly known methods. Further, the silver halide photographic materials used in the invention have to be sensitive to light (or radiation) emitted by an intensifying screen. Silver halide emulsions inherently have sensitivity to light or radiation in the range of blue to ultraviolet. For example, in the case of calcium tungstate a as phosphor contained in the screen, light emitted by a intensifying screen is in the range of blue to ultraviolet. In the case of an intensifying screen containing terbium-activated gadolinium oxysulfide phosphor, silver halide of the photographic material must be sensitized to green light region.

In silver halide photographic materials used in this invention, as afore-mentioned, the dye layer is preferably capable of being decolorized in the processing, and it is therefore preferred that the amount of binder used in the light sensitive layer provided on the dye layer is reduced as much as possible. Thus, the binder content of the light sensitive layer is preferably not more than 5 $g/m^2$, more preferably not more than 3 $g/m^2$ and not less than 1 $g/m^2$. Further, the silver content is preferably not more than 3 $g/m^2$ and more preferably not more than 2 $g/m^2$, and in this case, the lower limit thereof is preferably not less than 0.5 $g/m^2$. A protective layer comprising an aqueous soluble polymer such as gelatin is provided on the sublayer and on the light sensitive layer provided on both sides of the support. Silver halide photographic materials used in this invention can thus be obtained.

In silver halide photographic materials according to this invention, having a characteristic curve and exhibiting a reduced cross-over, it was proved that superior image quality and enhanced sensitivity could be achieved through the image forming method of this invention, by the use of a silver halide photographic material having sensitivity within a specific region in combination with an intensifying screen exhibiting a relatively high-sensitivity and a CTF (contrast transfer function) of not less than 0.79 at a spatial frequency of 1 line/mm and not less than 0.36 at a spatial frequency of 3 lines/mm. Thus it means that any combination of a photographic material and an intensifying screen is applicable but a specific combination thereof leads to enhanced image quality balanced with sensitivity. It is more preferred to

have a relatively high sensitive intensifying screen exhibiting an X-ray absorption of not less than 25% with respect to X-rays at 80 kVp, a CTF of not less than 0.79 at a spatial frequency of 1 line/mm and not less than 0.36 at a spatial frequency of 3 lines/mm, combined with a photographic material exhibiting a decrease in sensitivity which is counterbalanced with enhanced sensitivity of the screen.

Next, radiographic intensifying screens used in this invention will be described in detail. The radiographic intensifying screen used in the photographic combination according to this invention can be readily prepared by methods commonly known in the art to adjust the sensitivity to meet the requirements of this invention. Examples of intensifying screens are described in Research Disclosure item 18431, sect, IX.

The basic structure of the radiographic intensifying screen comprises a support on one side thereof having a phosphor layer. The phosphor layer is a layer comprising phosphor dispersed in a binder. Further, a transparent protective layer is provided on the opposite surface of the support to the phosphor layer, which protects the phosphor layer from chemical alteration or physical impact.

Phosphor for use in radiographic intensifying screens used in this invention is preferably a compound represented by the following formula:

$$M_{(w-n)}M'_nO_wX$$

wherein M is at least one of metallic yttrium, lanthanum, gadolinium, and ruthenium; M' is at least one of rare earth elements, preferably dysptosium, erbium, , europium, holmium, neodium, praseodymium, samarium, cerbium, terbium, thulium and ytterbium; X is an intermediate chalcogen (S, Se and Te) or a halogen; n is a number of 0.0002 to 0.2; w is 1 when X is a halogen and 2 when X is a chalcogen.

Examples of preferred phosphors for use in radiographic intensifying screens used in this invention include terbium activated rare earth acid sulfide phosphors[$Y_2O_2S$:Tb, $Gd_2O_2S$:Tb, $La_2O_2S$:Tb, $(Y,Gd)_2O_2S$:Tb, $(YGd)_{22}S$:Tb, Tm etc.]terbium activated rare earth oxyhalide phosphor (LaOBr:Tb, LaOBr:Tb, Tm, LaOCl:Tb, LaOCl:Tb, Tm, GdOBr: Tb, GdOCl:Tb, etc.), thulium activated rare earth oxyhalide phosphor, )LaOBr:Tm, LaOCl:Tm, etc.). Of the above mentioned phosphors, preferred phosphors used in the radiographic intensifying screen relating to this invention inclide, for example, terbium activated gadolinium acid sulfide (oxysulfide) phosphor. Terbium activated gadolinium oxysulfide phosphors are detailed in U.S. Patent No. 3,725,704.

Providing a phosphor layer on the support is conducted by the use of coating under ordinary pressure. Thus, phosphor particles and a binder are mixed in an appropriate solvent and dispersed to prepare a coating solution. The coating solution is coated on a support used for intensifying screens by means of doctor blade, roll coater or knife coater under ordinary pressure. Thereafter, a phosphor layer is provided by removing solvents or in such a manner that a coating solution is coated on a glass plate under ordinary pressure in advance, then, solvents are removed from the coat to form a thin phosphor containing resin coat and then the layer peeled from the temporary support and the peeled layer is adhered onto the support.

The radiographic intensifying screen used in this invention can be prepared according to the manner described above but is preferred to prepare the screen using a thermal plastic elastomer as binder by a compression treatment to enhance the filling ratio of the phosphor, as described below.

Sensitivity of a radiographic intensifying screen is basically dependent of the total emission of phosphor contained in the panel. The total emission depends on not only emission luminance of the phosphor but also the content of the phosphor. The more the phosphor content, the more absorption of X-rays, thereby leading to enhanced sensitivity and enhanced image quality (specifically, graininess). In cases where the phosphor content of the phosphor layer is constant, the closely packed phosphor results in reduced layer thickness, thereby minimizing diffusion of emission, caused by scattering and leading to relatively enhanced sharpness.

The radiographic intensifying screen is prepared preferably by a process of (a) forming a phosphor sheet comprising binder and phosphor and (b)putting the phosphor sheet on a support and adhering the phosphor sheet onto the support by compression at a temperature higher than a softening temperature or melting point of the binder resin.

First, step (a) will be described. The phosphor sheet forming a phosphor layer of the intensifying screen can be prepared by coating a coating composition of a phosphor homogeneously dispersed in a binder solution on a temporary support to form a phosphor sheet, drying and peeling the phosphor sheet from the temporary support. Thus, at first, a binder and phosphor particles are dispersed in an appropriate organic solvent to prepare a phosphor coating solution.

As a binder, a thermoplastic elastomer whose softening temperature or a melting point is 30 to 150°C is used singly or in combination with other binder polymers. The thermoplastic elastomer has elasticity at room temperature and has fluidity when heated. Therefore, it can prevent damage of the phosphor due to pressure in compression. As examples of a thermo-plastic elastomer, polystyrene, polyolefin, polyurethane, polyester, polyamide, polybutadiene,

ethylene vinyl acetate copolymer, poly vinyl chloride, natural rubbers, fluorine-containing rubbers, polyisoprene, chlorinated polyethylene, styrene-butadiene rubbers and silicone rubbers are cited. The component ratio of thermo-plastic elastomer in the binder is allowed to be 10 wt% or more and 100 wt% or less. However, it is desirable that the binder is composed of the thermo-plastic elastomer as much as possible, especially is composed of a thermo-plastic elastomer of 100 wt%.

As examples of a solvent for preparing a coating solution, lower alcohols such as methanol, ethanol, n-propanol and n-butanol; chlorine-containing hydrocarbons such as methylenechloride and ethylenechloride; ketones such as acetone, methylethylketone and methylisobutylketone; esters of lower fatty acids and lower alcohols such as methyl acetate, ethyl acetate and butyl acetate; ethers such as dioxane, ethyleneglycolmonoethylether and ethyleneglycolmonomethylether and their mixtures can be cited. The mixture ratio between the binder and the phosphor in the coating solution varies depending upon the characteristic of the radiographic intensifying screen and the kind of phosphor. Generally, the mixture ratio of the binder and the phosphor is from 1:1 to 1:100 (by weight), and preferably from 1:8 to 1:40 (by weight).

Various additives such as a dispersant for improving dispersing property of a phosphor in aforesaid coating solution and a plasticizer for improving binding force between a binder and a phosphor in the phosphor layer after being formed may be mixed. Examples of a dispersant used for the above-mentioned purpose include phthalic acid, stearic acid, caproic acid and lipophilic surfactants may be cited. Examples of a plasticizer include phosphates such as triphenyl phosphate, tricresyl phosphate and diphenyl phosphate; phthalates such as diethyl phthalate and dimethoxyethyl phthalate; ester glycols such as ethylphthalylethyl glycolate and butylphthalylbutyl glycolate; and polyesters of polyethylene glycols and aliphatic dibasic acids such as polyester of triethylene glycol and adipic acid and polyester between diethylene glycol and succinic acid are cited. Next, the coating layer is formed by coating the coating solution containing the phosphor and the binder prepared in the above-mentioned manner on the tentative support for forming a sheet uniformly. This coating operation can be conducted by the use of a conventional means such as a doctor blade method, a roll coater method and a knife coater method.

A material of the tentative support can be selected from glass, metal plate or conventional materials as a support for an intensifying screen of X-ray. Examples of such materials include plastic films such as cellulose acetate, polyester, polyethylene terephthalate, polyamide, polyimide, triacetate and polycarbonate, metallic sheets such as aluminium foil and aluminium alloy foil, an ordinary paper, baryta paper, resin-coated paper, pigment paper containing a pigment such as titanium dioxide, paper wherein polyvinyl alcohol is subjected to sizing, ceramic plates or sheets such as alumina, zirconia, magnesia and titania. A coating solution for forming the phosphor layer is coated on the tentative support and dried. Following this, the coating layer is peeled off from the tentative support so that the phosphor sheet which will be a phosphor layer of a radiographic intensifying screen is formed. Therefore, it is desirable that a mold-releasing agent is coated on the surface of the tentative support and that the phosphor sheet formed is easily peeled off from the tentative support.

Next, step (b) will be described. A support for the thus prepared phosphor sheet is prepared. This support can be selected from materials similar to those used for the temporary support.

In a conventional radiographic intensifying screen, in order to strengthen binding between a support and a phosphor layer and in order to improve sensitivity or image quality (sharpness and graininess) as the radiographic intensifying screen, it is known to coat a polymer substance such as gelatin as an adhesive layer on the surface of a support on the side of the phosphor layer or to provide thereon a light-reflection layer comprising a light-reflective substance such as titanium dioxide or a light-absorption layer comprising a light-absorptive substance such as carbon black. The support used in the present invention may be provided with each of the above-mentioned layer. The constitution may be arbitrarily selected depending upon the purpose and application of the desired radiographic intensifying screen. The phosphor sheet obtained through step a) is loaded on a support. Next, the phosphor sheet is stuck on the support while compressing it at a softening temperature or a melting point or higher of the binder.

In the above-mentioned manner, by the use of a method that compress the phosphor sheet without fixing it on the support in advance, the sheet can be spread thinly. Accordingly, it prevents damage of the phosphor. In addition, compared to a case wherein the sheet is fixed for being pressed, a higher phosphor filling rate can be obtained even with the same pressure. Examples of a compressor used for compressing processing of the present invention include conventional ones such as a calendar roll and a hot press. In compression processing by the use of the calendar roll, the phosphor sheet obtained through step a) is loaded on the support, and then, the sheet is passed through rollers heated to the softening temperature or the melting point of the binder or higher at a certain speed. However, a compressor used for the present invention is not limited thereto. Any compressing means can be used, provided that it can compress the sheet while heating it. The compression pressure is preferably 500 N/cm$^2$ or more.

In an ordinary radiographic intensifying screen, a transparent protective layer is provided for protecting the phosphor layer physically and chemically on the surface of the phosphor layer opposite to that being in contact with the support, as described before. Such a protective layer is preferably provided in the radiographic intensifying screen of the present invention. Layer thickness of the protective layer is ordinarily in a range from about 0.1 to 20 μm. The

transparent protective layer can be formed by a method that coats a solution prepared by dissolving a transparent polymer such as cellulose derivatives including cellulose acetate and nitro cellulose; and a synthetic polymer including polymethyl methacrylate, polyvinyl butylal, polyvinyl formal, polycarbonate, polyvinyl acetate, vinyl chloride-vinyl acetate copolymer on the surface of the phosphor layer. In addition, the transparent protective layer can also be formed by a method that forms a sheet for forming a protective layer such as a plastic sheet composed of polyethylene terephthalate, polyethylene naphthalate, polyethylene, polyvinylidene chloride or polyamide; and a protective layer forming sheet such as a transparent glass plate is formed separately and they are stuck on the surface of the phosphor layer by the use of an appropriate adhesive agent.

As a protective layer used for the radiographic intensifying screen of the present invention, a layer formed by a coating layer containing an organic solvent soluble fluorescent resin is preferable. As a fluorescent resin, a polymer of a fluorine-containing olefin (fluoro olefin) or a copolymer of a fluorine-containing olefin is cited. A layer formed by a fluorine resin coating layer may be cross-linked. When a protective layer composed of a fluorine resin is provided, dirt exuded from a film in contacting with other materials and an X-ray film is difficult to come into inside of the protective layer. Therefore, it has an advantage that it is easy to remove dirt by wiping. When an organic solvent soluble fluorescent resin is used as a material for forming a protective layer, it can be formed easily by coating a solution prepared by dissolving this resin in a suitable solvent and drying it. Namely, the protective layer is formed by coating the protective layer forming material coating solution containing the organic solvent soluble fluorine resin on the surface of fluorescent layer uniformly by the use of the doctor blade and by drying it. This formation of a protective layer may be conducted concurrently with the formation of the phosphor layer by the use of multilayer coating.

The fluorine resin is a homopolymer or copolymer of a fluorine containing olefin (also denoted as fluoroolefin). Its examples include polytetrafluoroethylene, polychlorotrifluoroethylene, polyvinyl fluoride, polyvinylidene fluoride, tetrafluoroethylene-hexafluoropropylene copolymer and fluoroolefin-vinyl ether copolymer. Though fluorine resins are insoluble in an organic solvent, copolymers of fluoroolefins as a copolymer component are soluble in an organic solvent depending upon other constituting units (other than fluoroolefin) of the copolymers. Therefore, the protective layer can be formed easily by coating a solution wherein the aforesaid resin is dissolved in a suitable solvent for preparing on the phosphor layer to be dried. Examples of the above-mentioned copolymers include fluoroolefin-vinyl ether copolymer. In addition, polytetrafluoroethylene and its denatured product are soluble in a suitable fluorine-containing organic solvent such as a perfluoro solvent. Therefore, they can form a protective layer in the same manner as in the copolymer containing the above-mentioned fluoroolefin as a copolymer component.

To the protective layer, resins other than the fluorine resin may be incorporated. A cross-linking agent, a hardener and an anti-yellowing agent may be incorporated. However, in order to attain the above-mentioned object sufficiently, the content of the fluorine resin in the protective layer is suitably 30 wt% or more, preferably 50 wt% or more and more preferably 70 wt% or more. Examples of resin incorporated in the protective layer other than the fluorine resin include a polyurethane resin, a polyacrylic resin, a cellulose derivative, polymethylmethacrylate, a polyester resin and an epoxy resin. The protective layer for the radiographic intensifying screen used in the present invention may be formed by either of an oligomer containing a polysiloxane skeleton or an oligomer containing a perfluoroalkyl group or by both thereof. The oligomer containing the polysiloxane skeleton has, for example, a dimethyl polysiloxane skeleton. It is preferable to have at least one functional group (for example, a hydroxyl group). In addition, the molecular weight (weight average) is preferably in a range from 500 to 100000, more preferably 1000 to 100000, especially more preferably 3000 to 10000. In addition, the oligomer containing the perfluoroalkyl group (for example, a tetrafluoroethylene group) preferably contains at least one functional group (for example, a hydroxyl group: -OH) in a molecule. Its molecular weight (weight average) is 500 to 100000, more preferably 1000 to 100000 and especially preferably 10000 to 100000. When an oligomer containing a functional group is used, cross-linking reaction occurs between the oligomer and a resin for forming a protective layer in forming the protective layer so that the oligomer is taken into a molecule structure of the layer-forming resin. Therefore, even when the X-ray conversion panel is used for a long time repeatedly or cleaning operation of the surface of the protective layer is carried out, the oligomer is not taken off from the protective layer. Therefore, the addition of the oligomer becomes effective for a long time so that use of the oligomer having a functional group becomes advantageous. The oligomer is contained in the protective layer preferably in an amount of 0.01 to 10 wt% and especially 0.1 to 2 wt%.

In the protective layer, perfluoro-olefin resin powder or silicone resin powder may be added. As the perfluoro-olefin resin powder or the silicone resin powder, those having an average particle size of preferably 0.1 to 10 μm, and more preferably 0.3 to 5 μm. The above-mentioned perfluoro-olefin resin powder or the silicone resin powder is added to the protective layer preferably in an amount of 0.5 to 30 wt% and more preferably 2 to 20 wt% and especially preferably 5 to 15 wt%.

The intensifying screen used in this invention preferably exhibits a relative high sensitivity and a CTF (contrast transfer function) of not less than 0.79 at a spatial frequency of 1 line/mm and not less than 0.36 at a spatial frequency of 3 lines/mm.

Further, when a cirved is formed by connecting points represented by the following spatial frequency and CTF

values in the coordinate of a spatial frequency (line/mm) as an abscissa and a contrast transfer function (CTF) as an ordinate, the intensifying screen used in this invention preferably exhibits CTF values higher than the curve within the entire spatial frequency region:

| line/mm | CTF |
|---|---|
| 0.00 | 1.00 |
| 0.25 | 0.950 |
| 0.50 | 0.905 |
| 0.75 | 0.840 |
| 1.00 | 0.790 |
| 1.25 | 0.720 |
| 1.50 | 0.655 |
| 1.75 | 0.595 |
| 2.00 | 0.535 |
| 2.50 | 0.430 |
| 3.00 | 0.360 |
| 3.50 | 0.300 |
| 4.00 | 0.255 |
| 5.00 | 0.180 |
| 6.00 | 0.130. |

Measurement and calculation of contrast transfer function of from the intensifying screen to the photographic material can be made by printing a rectangular chart onto a single-sided MRE, available Eastman Kodak Co.

The intensifying screen having such characteristics can be obtained by using the thermal plastic elastomer described above and subjected the phosphor layer to the compression treatment.

The protective layer of the intensifying screen is preferably a transparent synthetic resin layer coated on the phosphor layer and having a thickness of 5 µm or less. The use of a thick protective layer leads to shorten the distance between the intensifying screen and a silver halide emulsion and therefore enhance sharpness of the resulting X-ray photographic image.

In the photographic combination according to this invention, it is preferred to use a silver halide photographic material in which the front and rear light sensitive layers meet the afore-mentioned requirements regarding sensitivity and have substantially identical characteristics with each other, in combination with intensifying screens exhibiting the afore-mentioned characteristics on both sides (or on the front and rear side) of the photographic material. Alternatively, as described in U.S. Patent No. 4,710,637, the phosphor content of the front-sided screen may be reduced, compared to that of the rear-sided screen to enhance the balance between image sharpness and sensitivity.

To achieve a photographic combination having sensitivity acceptable in practical use and exhibiting superior radiographic image quality, the photographic material is combined with two sheets of radiographic intensifying screens so that a density of 1.0 is obtained as sensitivity of the photographic combination when exposed to X-rays of 0.5 to 1.5 mR, emitted by a three-phase X ray source at 80 kVp and processed in the developer solution afore-mentioned.

## EXAMPLES

The present invention will be further described based on examples but embodiments of the invention are by no means limited to these.

Example 1

Preparation of Photographic Emulsion

Preparation of seed grain emulsion

A seed grain emulsion-1 was prepared as follows.

| Solution A1 | |
|---|---|
| Low molecular weight gelatin (av. MW of 15,000) | 24.2 g |
| Water | 9657 ml |
| EO compound {HO- $[CH_2CH_2O]_m$-$[CH(CH_3)$-$CH_2O]_{17}$-$[CH_2CH_2O]_n$-H (m+n=5.7, M.W. of 1700, 10% ethanol solution) | 6.78 ml |
| Potassium bromide | 10.8 g |
| 10% Nitric acid | 114 ml |

| Solution B1 | |
|---|---|
| Aq. 2.5 mol/l silver nitrate solution | 2825 ml |

| Solution C1 | |
|---|---|
| Potassium bromide | 841 g |
| Water to make | 2825 ml |

Solution D1

Aq. 1.75 mol/l potassium bromide solution for adjusting silver electrode potential

Using a stirring mixer described in JP-B No. 58-58288 and 58-58289, solution B1 and C1, 464.3 ml of each were simultaneously added to solution A1 maintained at 42° C for a period of 1.5 min. to form nucleus grains. After stopping the addition of solution B1 and C1, the reaction mixture solution was heated to 60° C in 60 min., while the silver electrode potential was maintained at +8 mV and the pH was adjusted to 5.0 with 3% KOH and then solutions B1 and C1 were simultaneously added by the double jet addition at a flow rate of 55.4 ml/min. for a period of 42 min., while the silver electrode potential was maintained at +16 mV. The silver electrode potential was adjusted with solution D1 using a silver ion selection electrode, based a saturated silver-silver chloride reference electrode. After completion of addition, the pH was adjusted to 6.0 with 3% KOH.

The thus obtained seed grain emulsion was comprised of silver halide grains, in which at least 90% of the total grain projected area was accounted for by hexagonal tabular grains having a maximum adjacent edge ratio of 1.0 to 2.0 and exhibiting a mean grain thickness of 0.056 μm and a mean circular equivalent diameter of 0.595 μm, based on electron microscopic observation. A variation coefficient of grain thickness and a variation coefficient of spacing between twin planes were 40% and 42%, respectively.

Preparation of emulsion Em-1

Tabular silver halide grain emulsion Em-1 was prepared using seed grain emulsion-1 and four solutions.

| Solution A2 | |
|---|---|
| Ossein gelatin | 34.03 ml |
| EO compound (10% ethanol solution) | 2.25 ml |
| Seed grain emulsion-1 | 1.218 mol eq. |
| Water to make | 3150 ml |

| Solution B2 | |
|---|---|
| Potassium bromide | 1734 g |
| Water to make | 3644 ml |

| Solution C2 | |
|---|---|
| Silver nitrate<br>Water to make | 2478 g<br>4165 ml |

| Solution D2 | |
|---|---|
| Fine grain emulsion comprised of 3 wt% and silver iodide grains (av. size of 0.05 µm)* | 0.040 mol. eq. |

* The fine grain emulsion was prepared as follows. To 6.64 lit. of aqueous 5.0 wt% gelatin solution containing 0.06 mol potassium iodide, 7.06 mol silver nitrate and 7.06 mol potassium iodide, each of 2 lit. were added for 10 min., while the pH was maintained at 2.0 with nitric acid and the temperature was maintained at 40° C. After completion of grain formation, the pH was adjusted to 6.0.

To solution A2 in a reaction vessel maintained at 60° C with stirring were simultaneously added a part of solution B2 and a part of solution C2 by the double jet addition for 5 min., then, a half of the remaining solution B2 and a half of the remaining solution C2 were simultaneously added for 37 min., subsequently, a part of the remaining solution B2, a part of the remaining solution C2 and a total of solution D2 were added for 15 min., and finally, a total of the remaining solutions B2 and C2 was added, while the pH and pAg were maintained at 5.8 and 8.5, respectively. Addition of solutions B2 and C2 was acceleratingly increased so as to meet the critical grain growth rate. Thereafter, 95 ml of an aqueous 2N potassium thiocyanate solution was added and stirring was further done for 5 min.

Thereafter, the emulsion was cooled to 40° C and 1800 ml of an aqueous 13.8 wt% phenycarbamoyl-modified gelatin (substitution factor of 90%) solution, as a coagulant was added thereto and stirred fro 3 min. Then, the pH was adjusted to 4.6 with an aqueous 56 wt% acetic acid solution and stirred fro 3 min. After being allowed to stand for 20 min., the supernatant was decanted. Subsequently, 9.0 lit. of distilled water at 40° C was added and after being stirred and allowed to stand, the supernatant was decanted; 11.25 lit. of distilled water was added thereto and after being stirred and allowed to stand, the supernatant was decanted. The, an aqueous gelatin solution and an aqueous 10 wt% sodium carbonate solution were added thereto and after adjusting the pH to 5.80, the emulsion was dispersed at 50° C for 30 min. After dispersing, the pH and pAg were adjusted to 5.8 and 8.0 at 50° C, respectively. From electron microscopic observation, the thus obtained emulsion was comprised of tabular silver halide grains exhibiting a mean circular equivalent diameter of 0.84 µm, a mean grain thickness of 0.21 µm, a mean aspect ratio of 4.0, a mean sphere equivalent diameter of 0.61 µm and a variation coefficient of grain size distribution of 18.1%.

Preparation of emulsion Em-2

Emulsion Em-2 was prepared in a manner similar to Em-1, except that 1.64 mol eq. of seed grain emulsion-1 and 2.05 ml of solution A2 of EO compound were used and the pAg was varied to 8.7. From electron microscopic observation, the obtained emulsion was comprised of tabular silver halide grains exhibiting a mean circular equivalent diameter of 0.84 µm, a mean grain thickness of 0.16 µm, a mean aspect ratio of 5.3, a mean sphere equivalent diameter of 0.55 µm and a variation coefficient of grain size distribution of 21.2%.

Preparation of emulsion Em-3

Emulsion Em-3 was prepared in a manner similar to Em-1, except that 2.935 mol eq. of seed grain emulsion-1 and 1.80 ml of solution A2 of EO compound were used and the pAg was varied to 8.9. From electron microscopic observation, the obtained emulsion was comprised of tabular silver halide grains exhibiting a mean circular equivalent diameter of 0.84 µm, a mean grain thickness of 0.09 µm, a mean aspect ratio of 9.3, a mean sphere equivalent diameter of 0.46 µm and a variation coefficient of grain size distribution of 25.4%.

Preparation of emulsion Em-4

Emulsion Em-4 was prepared in a manner similar to Em-1, except that 2.15 ml of solution A2 of EO compound were used and the pAg was varied to 8.6. From electron microscopic observation, the obtained emulsion was comprised of tabular silver halide grains exhibiting a mean circular equivalent diameter of 0.96 µm, a mean grain thickness of 0.16 µm, a mean aspect ratio of 6.0, a mean sphere equivalent diameter of 0.61 µm and a variation coefficient of grain size distribution of 22.2%.

Preparation of metal-doped emulsions Em-5 to Em-8

Metal-doped emulsions Em-5 to Em-8 were prepared in a manner similar to Em-1 to Em-4, respectively, except that at the time of adding fine grain emulsion D2, potassium salt of SET-5, $K_3[Ir]CN)_6]$ of $8.2\times10^{-6}$ mol was added to solution B2. From electron microscopic observation of these metal-doped grains, no variation of grain shape was observed.

Chemical sensitization of emulsion

Succeedingly, emulsions Em-1 to Em-8 were each melted and maintained at 50° C with stirring. Then, spectral sensitizing dye (A) was added, in the form of a solid particle dispersion, to each of the emulsions in an amount shown in Table 1. After 30 min., a selenium sensitizer dispersion (triphenylphosphine selenide) of an amount shown in Table 1, and an aqueous mixture of 65 mg of ammonium thiocyanate, 4.6 mg of chloroauric acid and $5\times10^{-6}$ mol of sodium thiosulfate was added to the emulsion and after 40 min., the fine grain emulsion of silver iodide of $3.0\times10^{-3}$ mol was further added. Thereafter, stabilizer 4-hydroxy-1,3,3a,7-tetrazaindene of 200 mg was added to complete chemical sensitization and the emulsion was cooled. Each emulsion was thus chemically sensitized to an optimal level.

The solid particle dispersion of sensitizing dye (A) was prepared in accordance with the method described in JP-A 5-297496. Thus, a given amount of the sensitizing dye was added to water previously maintained at 27° C and stirred with a high-speed stirrer (dissolver) at a rate of 500 rpm for a period of 30 to 120 min.

Spectral sensitizing dye (A): 5,5'-dichloro-9-ethyl-3,3'-di-(sulfopropyl) oxacarbocyanine sodium salt anhydride.

The triphenylphosphine selenide dispersion was prepared as follows. Thus, 120 g of triphenylphosphine selenide was added to 30 kg of ethyl acetate at 50° C, dissolved with stirring, mixed with a solution containing 93 g of an aqueous 25 wt% sodium dodecybenzenesolfonate and dispersed at 50° C for 30 min., using a high-speed stirrer type dispersing machine provided with a 10 cm diameter dissolver at a dispersing-blade circumferential speed of 40 m/sec. Then, the dispersion mixture was stirred under reduced pressure until reached less than 0.3% by weight of remaining ethyl acetate. Thereafter, the dispersion was diluted with pure water to make the total amount of 80 kg. The thus prepared emulsions are shown in Table 1.

Table 1

| | Em-1 | Em-2 | Em-3 | Em-4 | Em-5 | Em-6 |
|---|---|---|---|---|---|---|
| Mean CED*1 | 0.84µm | 0.84µm | 0.84µm | 0.96µm | 0.84µm | 0.84µm |
| Mean SED*2 | 0.61µm | 0.55µm | 0.46µm | 0.61µm | 0.61µm | 0.55µm |
| Sensitizing dye (A) | 330mg | 360mg | 520mg | 330mg | 330mg | 360mg |
| Se Sensitizer | 3.2mg | 3.5mg | 5.1mg | 3.2mg | 3.2mg | 3.5mg |

| | Em-7 | Em-8 |
|---|---|---|
| Mean CED*1 | 0.84µm | 0.96µm |
| Mean SED*2 | 0.46µm | 0.61µm |
| Sensitizing dye (A) | 520mg | 330mg |
| Se Sensitizer | 5.1mg | 3.2mg |

*1: Circular equivalent diameter
*2: Sphere equivalent diameter

Preparation of Photographic Material Sample

Preparation of filter layer

On a 175 µm thick, blue-tinted polyethylene terephthalate support which was sub-coated with a dispersion of

10 wt.% copolymer comprised of 50 wt.% of glycidyl methacrylate, 10 wt.% of methyl methacrylate and 40 wt.% of methyl methacrylate, a filter layer having the following composition was coated so as to have coating amounts per $m^2$ of one side, as shown below.

| | |
|---|---|
| Solid particle dispersion of Dye (AH) | 25 $mg/m^2$ |
| Gelatin | 160 $mg/m^2$ |
| Latex (L) | 312 $mg/m^2$ |
| Poly(sodium styrenesulfonate) | 4.4 $mg/m^2$ |
| Top Side 300 (available from Perchem Asia Corp.) | 0.4 $mg/m^2$ |

On the support coated with the filter layer, the following coating solutions were simultaneously coated in the order of an emulsion layer and a protective layer, using a slide hopper type coater and dried to obtain a photographic material sample.

Emulsion layer coating solution

To each of the foregoing emulsions, the following additives were added.

| | |
|---|---|
| 1,1-Dimethylol-1-brom-1-nitromethane | 0.1 $mg/m^2$ |
| t-Butyl cathecol | 1.75 $mg/m^2$ |
| Polyvinyl pyrrolidine (M.W. 10,000) | 13.5 $mg/m^2$ |
| Poly(sodium styrenesulfonate) | 40 $mg/m^2$ |
| Trimethylol propane | 110 $mg/m^2$ |
| Ammonium 1,3-dihydroxybebzebe-4-sulfonate | 28 $mg/m^2$ |
| Latex (L) | 1.27 $mg/m^2$ |
| $n\text{-}C_4H_9OCH_2CH(OH)CH_2N(CH_2COOH)_2$ | 260 $mg/m^2$ |
| Thallium nitrate | 0.5 $mg/m^2$ |
| Compound (M) | 5 $mg/m^2$ |
| Compound (N) | 1.5 $mg/m^2$ |
| Colloidal silica (LUDOX AM, available from du'Pont Co., 0.013 μm) | 2.55 $mg/m^2$ |
| Dextran (av. M.W. 40,000) | 320 $mg/m^2$ |
| Gelatin | 1.2 $mg/m^2$ |

| Protective layer coating solution | |
|---|---|
| Gelatin | 0.8 $mg/m^2$ |
| Matting agent of polymethyl methacrylate (area-average particle size of 6.0 μm) | 27 $mg/m^2$ |
| Compound (P) | 50 $mg/m^2$ |
| Compound (S-1) | 4.4 $mg/m^2$ |
| Compound (S-2) | 185 $mg/m^2$ |
| Compound (S-3) | 48 $mg/m^2$ |
| Top Side 300 (available from Perchem Asia Corp.) | 0.6 $mg/m^2$ |

The coating amount is represented per $m^2$ on the one side and the silver coating amount was 1.6 mg per $m^2$ of one side.

Latex (L)

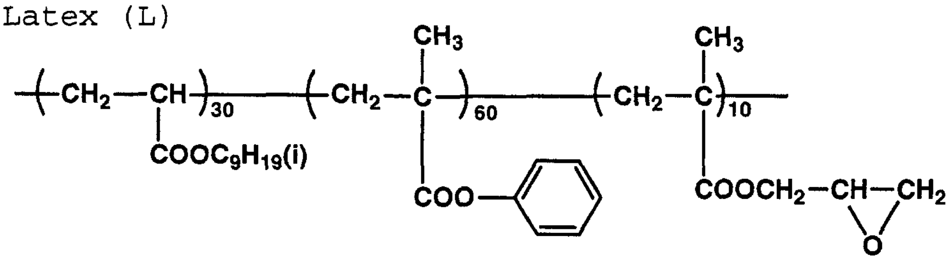

Compound (M)

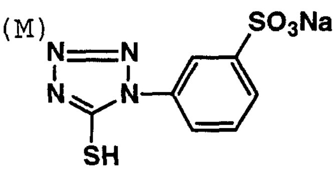

Compound (N)

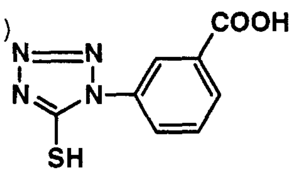

Compound (P)

$$\left( CH_2{=}CH-SO_2-CH_2CONHCH_2 \right)_2$$

Compound (S-1)

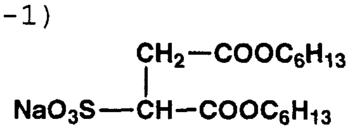

Compound (S-2)

$$C_7F_{15}CH_2\left(OCH_2CH_2\right)_{13}H$$

Compound (S-3)

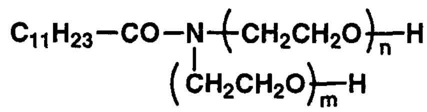

MW≒440

Dye (AH), solid particle dispersion

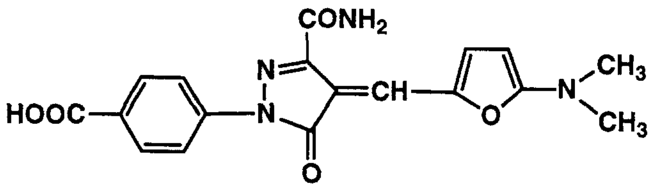

These coating solutions were coated on both side of the support using two slide hopper type coater at a coating speed of 120 m/min. so as to have a silver coverage of 1.6 g/$m^2$ of one side in the layer arrangement shown below and dried for a period of 2 min. 20 sec. to obtain Sample No. 1 to 8, in which each emulsion was singly contained in each emulsion layer, as shown in Table 2:

| Position | Layer |
|---|---|
| Upper layer | Protective layer |
| Intermediate layer | Emulsion layer |
| Lower layer | Filter layer |

Similarly, using emulsions Em-1 to Em-8, the following coating solutions were simultaneously coated on both sides of a 175 μm thick blue-tinted polyethylene terephthalate support in the order from the support of a filter layer, a lower emulsion layer, a upper emulsion layer and a protective layer, using a slide hopper type coater and dried to obtain photographic material Samples No. 9 to 27, as shown in Table 2.

| 1st Layer (Filter layer) | |
|---|---|
| Solid particle dispersion of Dye (AH) | 25 mg/$m^2$ |
| Gelatin | 160 mg/$m^2$ |
| Latex (L) | 300 mg/$m^2$ |
| Poly(sodium styrenesulfonate) | 4.4 mg/$m^2$ |
| Top Side 300 (available from Perchem Asia Corp.) | 0.4 mg/$m^2$ |

| 2nd Layer (Lower emulsion layer) | |
|---|---|
| 1,1-Dimethylol-1-brom-1-nitromethane | 0.05 mg/$m^2$ |
| t-Butyl cathecol | 1.0 mg/$m^2$ |
| Polyvinyl pyrrolidine (M.W. 10,000) | 7.0 mg/$m^2$ |
| Poly(sodium styrenesulfonate) | 20 mg/$m^2$ |
| Trimethylol propane | 55 mg/$m^2$ |
| Ammonium 1,3-dihydroxybebzebe-4-sulfonate | 14 mg/$m^2$ |
| Sodium 2-mercaptobenzimidazole-5-sulfonate | 5 mg/$m^2$ |
| $n-C_4H_9OCH_2CH(OH)CH_2N(CH_2COOH)_2$ | 130 mg/$m^2$ |
| Compound (M) | 2.5 mg/$m^2$ |
| Compound (N) | 0.5 mg/$m^2$ |
| Latex (L) | 0.6 mg/$m^2$ |
| Dextran | 150 mg/$m^2$ |
| Colloidal silica (LUDOX AM, available from du'Pont Co., 0.013 μm) | 1.3 mg/$m^2$ |

3rd Layer (Upper emulsion layer)

The layer composition was the same as the 2nd layer, provided that the total gelatin coating amount was 1.2 g/$m^2$, amounts of additives ware per one side and the contents of the lower emulsion layer and upper emulsion layer were calculated from the proportion of the silver content of each layer.

| 4th Layer (Lower protective layer) | |
|---|---|
| Gelatin | 0.2 mg/$m^2$ |
| Poly(sodium methyl methacrylate) (av. M.W. of 50,000)) | 30 mg/$m^2$ |
| Compound (K) | 15 mg/$m^2$ |

5th Layer (Upper protective layer)

| | |
|---|---|
| Gelatin | 0.4 mg/$m^2$ |
| Poly(methyl methacrylate) matting agent (av. particle size of 5.0 m) | 27 mg/$m^2$ |
| Colloidal silica (av. size of 0.014 μm) | 10 mg/$m^2$ |
| Compound (P) in an amount giving a swell ratio of 200% | |
| Compound (S-1) | 4.5 mg/$m^2$ |
| Compound (S-2) | 180 mg/$m^2$ |
| Compound (S-3) | 50 mg/$m^2$ |
| Top Side 300 (available from Perchem Asia Corp.) | 0.6 mg/$m^2$ |

Compound (K)

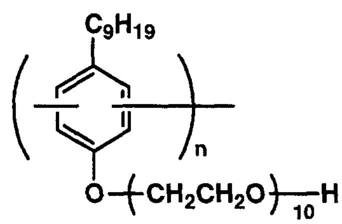

(mixture, n=2-5)

The coating amount is represented per $m^2$ on the one side and the silver coating amount was 1.6 mg per $m^2$ of one side.

Sample Nos. 1 through 8 and Sample Nos. 9 through 27 were each evaluated according to the following procedure and results thereof are shown in Tables 2 and 3.

Sensitometry

Photographic material samples were each sandwiched between radiographic intensifying screens SRO 250, (terbium activated gadolinium oxysulfide phosphor exhibiting the main emission at 545 nm, green light, available from Konica Corp.) and stepwise exposed to X-rays with varying the X-ray exposure amount by the exposure distance. X-ray bulb DRX-3724HD (available from Toshiba Electric Co., Ltd.) was used, in which the focus dimension was 0.6 mm and X-rays were generated through 3 mm thick aluminum-equivalent material including an aperture, using a tungsten target. X-rays which were generated by operating at a voltage of 80 kVp in a three-phase pulse generator and passed through a 7 cm thick water filter exhibiting an absorption equivalent to human body, was employed as a light source. Using a roller transport type automatic processor (SRX502, available from Konica Corp.), exposed photographic material samples were developed with developer solution D at 35° C and then fixed with fixer solution F, as described earlier, to obtain processed samples. The processed samples were each subjected to densitometry with visible light to obtain a characteristic curve. Sensitivity was represented by a relative value of the reciprocal of X-ray exposure necessary to give a density of 1.2, based on the sensitivity of sample 1 being 100. The thus obtained characteristic curve was differentiated to obtain a gamma curve of gamma (γ) vs. log E, where E is the exposure amount. From the gamma curve, the point gamma within the density region of 0.7 to 1.5 and the point gamma within the density region of 2.0 to 2.8 were determined. The results thereof are shown in Table 2.

Determination of Cross-over

Photographic material samples were each sandwiched between an intensifying screen (SRO250) and black opaque paper, and was exposed to X-rays from the screen side. The X-ray source was the same as used in the sensitometry and X-ray exposure was conducted by varying the exposure distance. Exposed samples were processed similarly to the sensitometry. The emulsion layer on one side of the support was peeled off and the emulsion layer on the other side was subjected to densitometry. The density of the emulsion layer on the side in contact with the intensifying screen was higher than that of the emulsion layer on the opposite side. The characteristic curve for the emulsion layer on each side was obtained and a mean value of the difference in log E (denoted as Δ log E) between densities of 0.5 and 1.0 corresponding to the straight line portion in the characteristic curve was determined. From the mean value, percent cross-over for each sample was calculated by using the following equation and results are shown in Table 2:

$$\text{Percent cross-over (\%)} = 100/(\text{antilog}\ (\Delta \log E) + 1).$$

Determination of CTF

Photographic material samples were each sandwiched between RSO250 intensifying screens and arranged at the position of 2 m from the X-ray source, and a rectangular chart for use in CTF measurement (molybdenum-based, thickness of 80 μm and a spatial frequency of 0 to 10 line/mm) was photographed. The X-ray source and photographic processing were the same as in the sensitometry. X-ray exposure was adjusted by an X-ray exposure time so as to give a density of 1.2 at the portion which was not shielded with molybdenum. The resulting processed samples were subjected to densitometry using a micro-densitometer, in which a density profile was measured at sampling intervals of 30 sec., using the aperture of a slit of 30 μm in the operation direction and 500 μm in the vertical direction thereto. This procedure was repeated 20 times to calculate an average value thereof to determine a density profile as a basis for determining CTF. Then, the peak of the square wave for a respective frequency of the density profile was detected to determine a density contrast for respective frequency. The measured values for a spatial frequency of 1 line/mm or 3 lines/mm are shown in Table 2.

Image evaluation through bone and stomach phantom

A foot phantom, produced by Kyoto Kagaku Co., Ltd. was employed and a micro-focus X-ray source, L6622-02 (available from Hamamatsu Photonics Co., Ltd.) was also employed at a focus dimension of 40 nm and a X-ray tube voltage of 120 kVp. The X-ray tube, phantom and a combination of a photographic material laminated with intensifying screens (SRO250) were arranged at the position of $R_1$ of 1 m and $R_2$ of 0.05 m to conduct radiography, in which $R_1$ was the distance between the X-ray source and the center of the phantom, and $R_2$ was the distance between the center of the phantom and the screen-film combination. Using a stomach phantom, produced by Kyoto Kagaku Co., Ltd. and the same X-ray source as used in the foot phantom, at a focus dimension of 40 nm and an X-ray tube voltage of 130 kVp, the X ray tube, phantom and a combination of a photographic material laminated with intensifying screens (SRO250) were arranged at the position of $R_1$ of 0.7 m and $R_2$ of 0.05 m to conduct radiography, in which $R_1$ was the distance between the X-ray source and the center of the phantom, and $R_2$ was the distance between the center of the phantom and the screen-film combination. Similarly to the sensitometry, exposed photographic material samples were processed with developer solution D and fixer solution F using automatic processor SRX502 over a period of 45 sec. (with development at 35° C for 13.5 sec.). Each sample was finished so as to exhibit a substantially identical density by adjusting the X-ray exposure time. Finished photographs were visually evaluated on a viewing box. Evaluation was made specifically with respect to clearness of bone skeleton and representation of soft tissue in foot phantom photographs, and representation of the fine structure of stomach walls and stomach body in stomach phantom photographs. The following criteria were used in the evaluation:

A: excellent
B: superior
C: minimally acceptable level for diagnosis
D: unacceptable level for diagnosis.

Foot phantom photographs and stomach phantom photographs were also evaluated with respect to graininess, based on the criteria, i.e., A: superior, B: good, C: fair, and D: poor.
Results are shown in Table 3.

Table 2

| Sample No. | Emulsion Upper (g/m²) | Emulsion Lower (g/m²) | Sensitivity (D=1.2) | Point Gamma D: 0.7-1.5 | Point Gamma D: 2.0-2.8 | Crossover (%) | CTF 1 line/mm | CTF 3 line/mm |
|---|---|---|---|---|---|---|---|---|
| 1 | Em−1 (1.6) | — | 100 | 2.4~3.3 | 1.2~2.7 | 12 | 0.79 | 0.42 |
| 2 | Em−2 (1.6) | — | 70 | 2.5~3.5 | 1.4~2.8 | 10 | 0.80 | 0.43 |
| 3 | Em−3 (1.6) | — | 35 | 2.6~3.6 | 1.4~2.9 | 8 | 0.80 | 0.44 |
| 4 | Em−4 (1.6) | — | 110 | 2.6~3.6 | 1.4~3.0 | 12 | 0.79 | 0.42 |
| 5 | Em−5 (1.6) | — | 105 | 2.4~3.3 | 1.2~2.7 | 12 | 0.79 | 0.42 |
| 6 | Em−6 (1.6) | — | 75 | 2.5~3.5 | 1.4~2.8 | 10 | 0.80 | 0.43 |
| 7 | Em−7 (1.6) | — | 38 | 2.6~3.6 | 1.4~2.9 | 8 | 0.80 | 0.44 |
| 8 | Em−8 (1.6) | — | 120 | 2.6~3.6 | 1.4~3.0 | 12 | 0.79 | 0.42 |
| 9 | Em−1 (0.8) | Em−2 (0.8) | 74 | 1.9~2.9 | 1.4~1.9 | 11 | 0.80 | 0.44 |
| 10 | Em−1 (0.8) | Em−3 (0.8) | 65 | 1.9~2.9 | 1.5~1.8 | 10 | 0.80 | 0.43 |
| 11 | Em−1 (0.8) | Em−4 (0.8) | 105 | 2.3~3.3 | 1.4~2.9 | 12 | 0.77 | 0.41 |
| 12 | Em−1 (0.8) | Em−5 (0.8) | 107 | 2.1~3.1 | 1.4~3.0 | 12 | 0.77 | 0.41 |
| 13 | Em−1 (0.8) | Em−6 (0.8) | 88 | 1.9~2.9 | 1.4~1.7 | 11 | 0.80 | 0.44 |
| 14 | Em−1 (0.8) | Em−7 (0.8) | 70 | 1.9~2.9 | 1.5~1.7 | 10 | 0.80 | 0.43 |
| 15 | Em−1 (0.8) | Em−8 (0.8) | 110 | 2.1~3.1 | 1.5~3.2 | 12 | 0.75 | 0.40 |
| 16 | Em−2 (0.8) | Em−3 (0.8) | 53 | 2.0~2.9 | 1.4~1.7 | 8 | 0.80 | 0.45 |
| 17 | Em−2 (0.8) | Em−7 (0.8) | 55 | 2.0~2.9 | 1.4~1.7 | 8 | 0.80 | 0.45 |
| 18 | Em−6 (0.8) | Em−3 (0.8) | 58 | 2.0~2.9 | 1.4~1.7 | 8 | 0.80 | 0.45 |
| 19 | Em−6 (0.8) | Em−7 (0.8) | 60 | 2.0~2.9 | 1.4~1.7 | 8 | 0.80 | 0.45 |
| 20 | Em−4 (0.8) | Em−2 (0.8) | 90 | 2.2~2.9 | 1.4~1.7 | 11 | 0.80 | 0.44 |
| 21 | Em−4 (0.8) | Em−3 (0.8) | 73 | 2.2~2.9 | 1.4~1.8 | 10 | 0.80 | 0.43 |
| 22 | Em−8 (0.8) | Em−2 (0.8) | 95 | 2.3~2.9 | 1.4~1.7 | 11 | 0.80 | 0.44 |
| 23 | Em−8 (0.8) | Em−3 (0.8) | 80 | 2.3~2.9 | 1.4~1.8 | 10 | 0.80 | 0.43 |
| 24 | Em−4 (0.8) | Em−6 (0.8) | 93 | 2.2~2.9 | 1.4~1.7 | 11 | 0.80 | 0.44 |
| 25 | Em−4 (0.8) | Em−7 (0.8) | 74 | 2.2~2.9 | 1.4~1.9 | 10 | 0.80 | 0.43 |
| 26 | Em−8 (0.8) | Em−6 (0.8) | 100 | 2.3~2.9 | 1.4~1.7 | 11 | 0.80 | 0.44 |
| 27 | Em−8 (0.8) | Em−7 (0.8) | 80 | 2.3~2.9 | 1.4~1.9 | 10 | 0.80 | 0.43 |

Table 3

| Sample No. | Object Distance | | | Bone Evaluation | | Stomach Evaluation | | Graininess |
|---|---|---|---|---|---|---|---|---|
| | $R_1$(m) | | $R_2$(m) | Skeleton | Soft Tissue | Wall | Body | |
| | bone | stomiach | | | | | | |
| 1 | 1.0 | 0.7 | 0.05 | C | C | C | C | C |
| 2 | 1.0 | 0.7 | 0.05 | C | C | B | C | C |
| 3 | 1.0 | 0.7 | 0.05 | - | - | - | - | C |
| 4 | 1.0 | 0.7 | 0.05 | C | C | B | D | D |
| 5 | 1.0 | 0.7 | 0.05 | C | C | C | C | C |
| 6 | 1.0 | 0.7 | 0.05 | C | C | B | C | C |
| 7 | 1.0 | 0.7 | 0.05 | - | - | - | - | C |
| 8 | 1.0 | 0.7 | 0.05 | C | C | B | D | D |
| 9 | 1.0 | 0.7 | 0.05 | C | B | C | B | C |
| 10 | 1.0 | 0.7 | 0.05 | C | B | C | B | C |
| 11 | 1.0 | 0.7 | 0.05 | C | B | C | B | D |
| 12 | 1.0 | 0.7 | 0.05 | C | B | C | B | C |
| 13 | 1.0 | 0.7 | 0.05 | C | B | C | B | C |
| 14 | 1.0 | 0.7 | 0.05 | C | B | C | B | C |
| 15 | 1.0 | 0.7 | 0.05 | C | B | C | B | D |
| 16 | 1.0 | 0.7 | 0.05 | C | B | C | B | C |
| 17 | 1.0 | 0.7 | 0.05 | C | B | C | B | C |
| 18 | 1.0 | 0.7 | 0.05 | C | B | C | B | C |
| 19 | 1.0 | 0.7 | 0.05 | C | B | C | B | C |
| 20 | 1.0 | 0.7 | 0.05 | C | B | C | B | C |
| 21 | 1.0 | 0.7 | 0.05 | C | B | C | B | C |
| 22 | 1.0 | 0.7 | 0.05 | C | B | C | B | C |
| 23 | 1.0 | 0.7 | 0.05 | C | B | C | B | C |
| 24 | 1.0 | 0.7 | 0.05 | C | B | C | B | C |
| 25 | 1.0 | 0.7 | 0.05 | C | B | C | B | C |
| 26 | 1.0 | 0.7 | 0.05 | C | B | C | B | C |
| 27 | 1.0 | 0.7 | 0.05 | C | B | C | B | C |

Using Sample Nos. 1 to 27, radiographing tests were done, in which distance $R_2$ was changed from 0.05 m to 0.7 m, provided that in Sample Nos. 9,10, 13 and 14, $R_2$ was 1.0 m. Results are shown in Table 4.

Table 4

| Sample No. | Object Distance | | | Bone Evaluation | | Stomach Evaluation | | Graininess | Remark |
|---|---|---|---|---|---|---|---|---|---|
| | $R_1$(m) | | $R_2$(m) | Skeleton | Soft Tissue | Wall | Body | | |
| | bone | stomach | | | | | | | |
| 1 | 1.0 | 0.7 | 0.7 | C | C | C | C | C | Comp. |
| 2 | 1.0 | 0.7 | 0.7 | C | C | B | C | B | Comp. |
| 3 | 1.0 | 0.7 | 0.7 | - | - | - | - | B | Comp. |
| 4 | 1.0 | 0.7 | 0.7 | C | C | B | D | C | Comp. |
| 5 | 1.0 | 0.7 | 0.7 | C | C | C | C | C | Comp. |
| 6 | 1.0 | 0.7 | 0.7 | C | C | B | C | B | Comp. |
| 7 | 1.0 | 0.7 | 0.7 | - | - | - | - | B | Comp. |
| 8 | 1.0 | 0.7 | 0.7 | C | C | B | D | C | Comp. |
| 9 | 1.0 | 0.7 | 0.7 | A | A | A | A | A | Inv. |
| 10 | 1.0 | 0.7 | 0.7 | A | A | A | A | A | Inv. |
| 11 | 1.0 | 0.7 | 0.7 | C | B | C | B | C | Comp. |
| 12 | 1.0 | 0.7 | 0.7 | C | B | C | B | B | Comp. |
| 13 | 1.0 | 0.7 | 0.7 | A | A | A | A | A | Inv. |
| 14 | 1.0 | 0.7 | 0.7 | A | A | A | A | A | Inv. |
| 15 | 1.0 | 0.7 | 0.7 | C | B | C | B | B | Comp. |
| 16 | 1.0 | 0.7 | 0.7 | A | A | A | A | A | Inv. |
| 17 | 1.0 | 0.7 | 0.7 | A | A | A | A | A | Inv. |
| 18 | 1.0 | 0.7 | 0.7 | A | A | A | A | A | Inv. |
| 19 | 1.0 | 0.7 | 0.7 | A | A | A | A | A | Inv. |
| 20 | 1.0 | 0.7 | 0.7 | A | A | A | A | A | Inv. |
| 21 | 1.0 | 0.7 | 0.7 | A | A | A | A | A | Inv. |
| 22 | 1.0 | 0.7 | 0.7 | A | A | A | A | A | Inv. |
| 23 | 1.0 | 0.7 | 0.7 | A | A | A | A | A | Inv. |
| 24 | 1.0 | 0.7 | 0.7 | A | A | A | A | A | Inv. |
| 25 | 1.0 | 0.7 | 0.7 | A | A | A | A | A | Inv. |
| 26 | 1.0 | 0.7 | 0.7 | A | A | A | A | A | Inv. |
| 27 | 1.0 | 0.7 | 0.7 | A | A | A | A | A | Inv. |

## Claims

**1.** A silver halide photographic light sensitive material which is combined with at least an intensifying screen to form a photographic combination for use in a radiographic imaging system for making a radiograph by a radiography apparatus, wherein the photographic material comprises a support having a light sensitive silver halide emulsion layer on each of both sides of the support, exhibiting a cross-over of not more than 15%, a point gamma of 1.8 to 3.0 within the range of a density of 0.7 to 1.5 on a characteristic curve and a point gamma of 1.2 to 2.0 within the range of a density of 2.0 to 2.8 on the characteristic curve when the photographic combination is exposed to X-ray and subjected to processing (A), wherein the processing (A) is conducted using a roller transport type automatic

processor and the following developer solution (D) and fixed solution (F) at a developing temperature of 35° C for a developing time of 12 to 15 sec. and at a fixing temperature of 33° C for a fixing time of 7 to 13 sec.:

| Developer solution D | |
|---|---|
| Water | 800 ml |
| Potassium hydroxide | 17.1 g |
| Potassium sulfite | 65.7 g |
| Potassium metabisulfite | 6.7 g |
| Hydroquinone | 27.0 g |
| Potassium carbonate | 9.0 g |
| Trisodium ethylenediaminetetraacetate (35% aqueous solution) | 8.0 g |
| Triethylene glycol | 11.8 g |
| 5-nitroindazole | 0.03 g |
| Acetic acid (80% aqueous solution) | 16.0 g |
| 1-Phenyl-3-pyrazolidone | 1.2 g |
| 1-Phenyl-5-mercaptotetrazole | 0.015 g |
| 5-Methylbenzotriazole | 0.024 g |
| Glutar aldehyde (50% aqueous solution) | 8.6 g |
| Potassium bromide | 2.6 g |
| Water to make | 1 lit. |

in which the pH is adjusted to 10.33; and

| Fixer solution F | |
|---|---|
| Water | 130 ml |
| Ammonium thiosulfate (75% weight/volume) | 245.5 g |
| Sodium sulfite | 11.7 g |
| Sodium hydroxide | 6.56 g |
| Sodium gluconate | 2.02 g |
| Citric acid monohydrate | 2.40 g |
| Acetic acid (80% aqueous solution) | 22.5 g |
| Aluminum sulfate | 9.8 g |
| 75% Sulfuric acid | 5.73 g |
| Water to make | 1 lit. |

in which the pH is adjusted to 4.18.

**2.** The photographic material of claim 1, wherein the light sensitive silver halide emulsion layer comprises a first silver halide emulsion layer containing a first tabular silver halide grains and a second silver halide emulsion layer containing a second silver halide tabular grains, the first and the second tabular grains are substantially identical in average circular equivalent diameter and different in average thickness.

**3.** The photographic material of claim 2, wherein a ratio of a sensitivity of the first emulsion layer to that of the second emulsion layer is 0.3 to 0.7.

**4.** The photographic material of claim 2 or 3, wherein the first emulsion layer is provided on the support and further thereon, the second layer is provided.

**5.** The photographic material of claim 2, 3 or 4, wherein the average circular equivalent diameter is 0.2 to 6.0 µm.

**6.** The photographic material of claim 2, 3, 4 or 5, wherein the tabular grains exhibit an average aspect ratio of circular equivalent diameter to thickness is 2.0 to 20.0.

**7.** The photographic material of any one of preceding claims, wherein the support is a transparent support and layer

arrangements on opposite sides of the support being identical with each other.

**8.** An radiographic imaging system for making a radiograph by a radiography apparatus using a photographic combination of a silver halide photographic light sensitive material in combination with at least an intensifying screen, the photographic material comprising a support having a light sensitive silver halide emulsion layer on each of both sides of the support, wherein the photographic material exhibits a cross-over of not more than 15%, a point gamma of 1.8 to 3.0 within the range of a density of 0.7 to 1.5 on a characteristic curve and a point gamma of 1.2 to 2.0 within the range of a density of 2.0 to 2.8 on the characteristic curve when the photographic combination is exposed to X-ray, and wherein the radiography apparatus conducts making a radiograph under the condition that a distance between a focal point of an X-ray tube and the photographic combination is 1.0 to 3.0 m and a distance between an object and the photographic combination is 0.2 to 1.5 m.

**9.** The system of claim 8, wherein the photographic material is positioned between intensifying screens.

**10.** The system of claim 8 or 9, wherein the distance between the focal point of an X-ray tube and the photographic combination is 1.5 to 2.5 m.

**11.** The system of claim 8, 9 or 10, wherein the distance between the object and the photographic combination is 0.5 to 1.2 m.

**12.** The system of any one of claims 8 to 11, wherein the intensifying screen exhibits a CTF value of not less than 0.79 at a spatial frequency of 1 line/mm and a CTF value of not less than 0.36 at a spatial frequency of 3 lines/mm.

**13.** The system of any one of claims 8 to 12, wherein in the photographic combination, the photographic material is combined with two intensifying screens so as to give a density of 1.0 when the photographic combination is exposed to 0.5 to 1.5 mR of X-ray produced by a three phase X-ray source operated at 80 kVp and subjected to processing (A), and wherein the processing (A) is conducted using a roller transport type automatic processor and the following developer solution (D) and fixed solution (F) at a developing temperature of 35° C for a developing time of 12to 15 sec. and at a fixing temperature of 33° C for a fixing time of 7 to 13 sec.:

| Developer solution D | |
|---|---|
| Water | 800 ml |
| Potassium hydroxide | 17.1 g |
| Potassium sulfite | 65.7 g |
| Potassium metabisulfite | 6.7 g |
| Hydroquinone | 27.0 g |
| Potassium carbonate | 9.0 g |
| Trisodium ethylenediaminetetraacetate (35% aqueous solution) | 8.0 g |
| Triethylene glycol | 11.8 g |
| 5-nitroindazole | 0.03 g |
| Acetic acid (80% aqueous solution) | 16.0 g |
| 1-Phenyl-3-pyrazolidone | 1.2 g |
| 1-Phenyl-5-mercaptotetrazole | 0.015 g |
| 5-Methylbenzotriazole | 0.024 g |
| Glutar aldehyde (50% aqueous solution) | 8.6 g |
| Potassium bromide | 2.6 g |
| Water to make | 1 lit. |

in which the pH is adjusted to 10.33; and

| Fixer solution F | |
|---|---|
| Water | 130 ml |
| Ammonium thiosulfate (75% weight/volume) | 245.5 g |
| Sodium sulfite | 11.7 g |

(continued)

| Fixer solution F | |
|---|---|
| Sodium hydroxide | 6.56 g |
| Sodium gluconate | 2.02 g |
| Citric acid monohydrate | 2.40 g |
| Acetic acid (80% aqueous solution) | 22.5 g |
| Aluminum sulfate | 9.8 g |
| 75% Sulfuric acid | 5.73 g |
| Water to make | 1 lit. |

in which the pH is adjusted to 4.18.

**14.** The system of any one of claims 8 to 13, wherein the photographic material is one as claimed in any one of claims 1 to 7.

FIG. 1

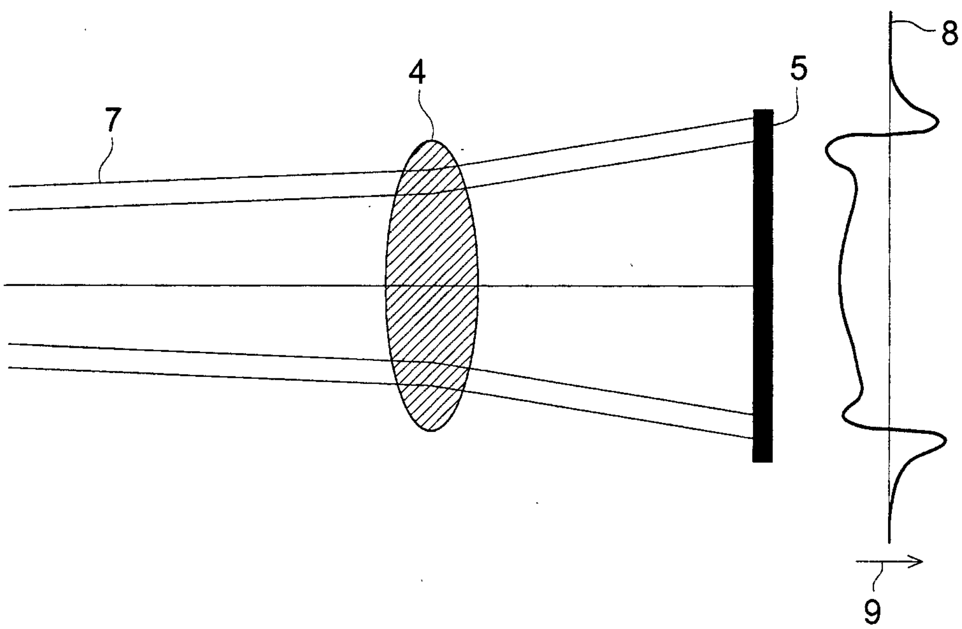

FIG. 2

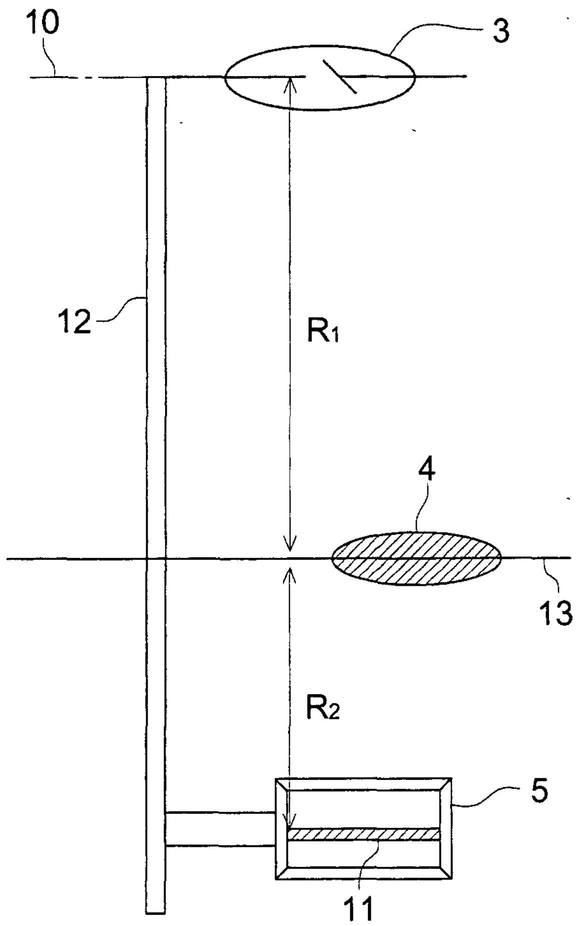

FIG. 3

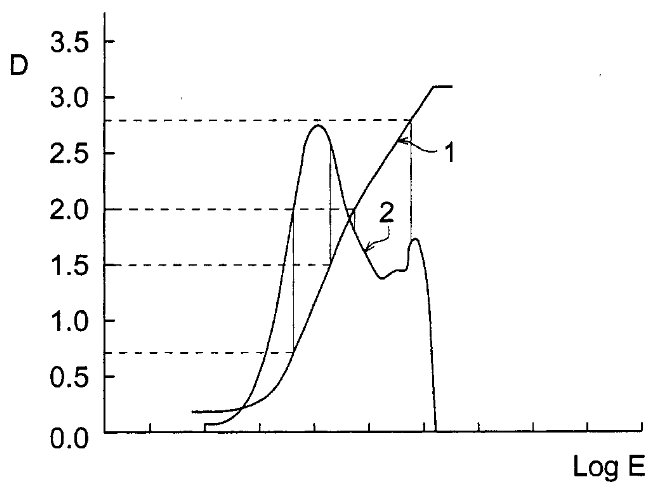

European Patent Office

# EUROPEAN SEARCH REPORT

Application Number
EP 01 10 5308

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 5 462 832 A (IWASAKI NOBUYUKI) 31 October 1995 (1995-10-31) * column 8, line 9 - line 40; claims; figure * | 1-14 | G03C5/17 G03C5/02 G03C5/26 |
| X | PATENT ABSTRACTS OF JAPAN vol. 2000, no. 01, 31 January 2000 (2000-01-31) & JP 11 295829 A (KONICA CORP), 29 October 1999 (1999-10-29) * abstract * | 1 | |
| A | US 5 460 916 A (IWASAKI NOBUYUKI) 24 October 1995 (1995-10-24) * the whole document * | 1-14 | |

TECHNICAL FIELDS SEARCHED (Int.Cl.7)
G03C

The present search report has been drawn up for all claims

| Place of search | Date of completion of the search | Examiner |
|---|---|---|
| THE HAGUE | 30 July 2001 | Buscha, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT ON EUROPEAN PATENT APPLICATION NO.** EP 01 10 5308

This annex lists the patent family members relating to the patent documents cited in the above-mentioned European search report.
The members are as contained in the European Patent Office EDP file on
The European Patent Office is in no way liable for these particulars which are merely given for the purpose of information.

30-07-2001

| Patent document cited in search report | Publication date | Patent family member(s) | Publication date |
|---|---|---|---|
| US 5462832 A | 31-10-1995 | JP 3051605 B | 12-06-2000 |
| | | JP 7028203 A | 31-01-1995 |
| JP 11295829 A | 29-10-1999 | NONE | |
| US 5460916 A | 24-10-1995 | JP 3051595 B | 12-06-2000 |
| | | JP 6332088 A | 02-12-1994 |

EPO FORM P0459

For more details about this annex : see Official Journal of the European Patent Office, No. 12/82